# EUROPÄISCHE PATENTSCHRIFT

(11) **EP 1 414 346 B1**
(45) Veröffentlichungstag und Bekanntmachung des Hinweises auf die Patenterteilung: **25.04.2007**
(21) Anmeldenummer: 02758117.2
(22) Anmeldetag: 24.07.2002
(51) Int. Cl.: A61B 5/22

(54) **VORRICHTUNG FÜR DIE UNTERSUCHUNG DER MUSKELKONTRAKTION**
DEVICE FOR EXAMINATION OF THE MOTOR SYSTEM OF THE HUMAN OR ANIMAL BODY
DISPOSITIF POUR EXAMINER LE SYSTEME MOTEUR DU CORPS HUMAIN OU ANIMAL

(30) Priorität: 26.07.2001 DE 10136310
(43) Veröffentlichungstag der Anmeldung: 06.05.2004
(73) Patentinhaber: Rahe-Meyer, Niels, 30177 Hannover (DE)
(72) Erfinder: Rahe-Meyer, Niels, 30177 Hannover (DE)
(74) Vertreter: Chambosse, Hans-Joachim
(86) Internationale Anmeldenummer: PCT/DE2002/002717
(87) Internationale Veröffentlichungsnummer: WO 2003/011137

(56) Entgegenhaltungen:
- EP-A- 0 232 507
- EP-A- 0 283 387
- WO-A-98/53740
- SINCLAIR P J ET AL: "Pedal forces produced during neuromuscular electrical stimulation cycling in paraplegics" CLINICAL BIOMECHANICS, BUTTERWORTH SCIENTIFIC LTD, GUILDFORD, GB, Bd. 11, Nr. 1, 1996, Seiten 51-57, XP004040298 ISSN: 0268-0033
- BOURBONNAIS D ET AL: "STATIC DYNAMOMETER FOR THE MEASUREMENT OF MULTIDIRECTIONAL FORCES EXERTED BY THE THUMB" MEDICAL AND BIOLOGICAL ENGINEERING AND COMPUTING, PETER PEREGRINUS LTD. STEVENAGE, GB, Bd. 29, Nr. 4, 1. Juli 1991 (1991-07-01), Seiten 413-418, XP000208767 ISSN: 0140-0118

## Beschreibung

Die Erfindung betrifft eine Vorrichtung zur Untersuchung des motorischen Systems des menschlichen und tierischen Körpers und insbesondere der Funktions- und Leistungsfähigkeit der Muskulatur nach dem Oberbegriff des Patentanspruchs 1. Die Muskelfunktion ist für das Individuum, insbesondere für den Menschen, eine notwendige Bedingung, um mit der Außenwelt Kontakt aufzunehmen und sie zu beeinflussen. Es ist deshalb von großer Bedeutung, das motorische System untersuchen zu können und dabei objektive und zuverlässige Messungen durchführen zu können und Ergebnisse von größtmöglicher Genauigkeit zu erzielen. Dies ist beispielsweise von erheblicher Bedeutung zur Diagnose und zur Beobachtung des Verlaufs von Erkrankungen des Bewegungsapparats und dabei namentlich von Muskelerkrankungen oder von Erkrankungen, die sekundär mit einer Beeinflussung der Funktionsfähigkeit des motorischen Systems verbunden sind, ferner für die Beobachtung des Verlaufs von Rehabilitationsprogrammen oder Trainingsprogrammen oder für die Kontrolle der Dosierung von muskelwirksamen Medikamenten. Von besonderer Wichtigkeit ist dafür, die Untersuchungen am jeweiligen Individuum und desselben Bereichs des Körpers mit identischen Bedingungen wiederholbar zu machen, weil erst dadurch Objektivität, Reliabilität und Validität der Ergebnisse erreicht wird.

Es sind zwar verschiedene Vorrichtungen und Verfahren für Muskeltests und die Messung bzw. die Ermittlung von Werten bei Bewegungsvorgängen und Muskelkräften bekannt. Sie haben jedoch neben anderen den erheblichen Nachteil, daß eine individuelle Einpassung der zu untersuchenden Körperbereiche, insbesondere der Glieder, der Untersuchungsperson nicht möglich ist und zudem keine, jedenfalls keine ausreichend zuverlässige, Fixierung dieser Bereiche erfolgt. Außerdem sind bei den bekannten Vorrichtungen keine Mittel für einstellbare Muskellängenbedingungen und ebensowenig Mittel für die Begrenzung von Muskellängenveränderungen vorhanden.

Aus der EP 0232507 B1 des Anmelders ist eine Vorrichtung bekannt, mit der der Bewegungsapparat durch künstliche Stimulationen und unterschiedliche Lasten beeinflußt wird und nachfolgend Nerven- und Muskelfunktionen gemessen werden. Diese Vorrichtung hat bereits wesentliche Nachteile der zuvor bekannten Vorrichtungen vermieden und sich im Einsatz an Gesunden und Kranken bewährt. Gleichwohl haben sich noch Nachteile gezeigt. Insbesondere ist es mit dieser vorbekannten Vorrichtung nicht möglich, die Untersuchungsbedingungen soweit zu standardisieren und zu objektivieren bzw. den Untersuchungsgegenstand und die Untersuchungsergebnisse bei einer ersten Untersuchung eines Individuums so festzulegen und von Fehlerquellen zu befreien, daß die sich ergebenden Meßergebnisse den Funktionszustand der Muskelmechanik des Untersuchten absolut charaterisieren und wiederholte vergleichende Untersuchungen zu ermöglichen. Namentlich ist bei dieser früheren Erfindung noch nicht möglich, die Muskellänge bei wiederholten Untersuchungen und Messungen immer gleich einzustellen. Auch ist die Kraftquelle nach der EP 0232507 B1 nicht konstant, nicht leistungsfähig und nicht wartungsfrei genug, um Störungen bzw. Ungenauigkeiten des Experimentalablaufs auszuschließen.

Aufgabe der Erfindung ist es daher, eine Vorrichtung zur Untersuchung des gesamten motorischen Systems des menschlichen und des tierischen Körpers zu schaffen, durch die wiederholbare und definierbare konstante Experimentaleingriffe und wiederholbare, absolute Messungen am motorischen System und insbesondere an Muskeln ermöglicht werden, indem insbesondere wiederholbare, einstellbare, zugleich aber variable Muskellängenbedingungen durch individuell anpassbare Verschienungseinrichtungen, Anschläge und Kraftbegrenzer sowie durch geeignete Meßeinrichtungen und Positionsbestimmungselemente bewirkt werden.

Diese Aufgabe wird im wesentlichen durch die kennzeichnenden Merkmale des Patentanspruchs 1 gelöst. Weiterbildungen und vorteilhafte Ausgestaltungen sind Gegenstand der weiteren Patentansprüche.

Nachfolgend werden Ausführungsbeispiele der Erfindung anhand von Zeichnungen erläutert. Es zeigen:
- Fig. 1:: die erfindungsgemäße Untersuchungsvorrichtung in Seitenansicht mit abgenommener Gehäusewand mit einer Ausführungsform der Auflage- und Fixierungseinrichtung für Hand und Handgelenk (oberer Bereich), Achsanschlag der Hauptgeräteachse mit vorderem und hinterem Rotations. anschlag (mittlerer Bereich) sowie Kraftquelle in Form von Drehmagneten mit Verstellungsvorrichtung (unterer Bereich);
- Fig. 2:: die Untersuchungsvorrichtung gemäß Fig. 1 in Frontalansicht mit abgenommener Verkleidung und als Längsschnitt im Bereich der Hauptgeräteachse;
- Fig. 3:: als Detail von Fig. 1 den Bereich des Achsanschlags mit vorderem Rotationsanschlag und Verstellmotor in Aufsicht von oben an der Schnittstelle B/B;
- Fig. 4:: als Detail von Fig. 1 den Bereich des Achsanschlags mit hinterem Rotationsanschlag und Verstellmotor in Aufsicht von oben an der Schnittstelle A/A;
- Fig. 5:: die Untersuchungsvorrichtung mit einer abgewandelten Auflage- und Fixierungseinrichtung für Arm und Hand sowie Kraftquelle in Form eines Elektromotors (Hallmotors) in Seitenansicht mit abgenommener Seitenwand;
- Fig. 6:: die abgewandelte Auflage- und Fixierungseinrichtung gemäß Fig. 5 jedoch von rechts, in der Aufsicht mit eingelegtem und fixiertem Arm-/Handbereich;
- Fig. 7:: die abgewandelte Auflage- und Fixierungseinrichtung der Untersuchungsvorrichtung wie in Fig. 6 jedoch mit eingelegtem rechten Arm und rechter Hand;
- Fig. 8:: die Auflage- und Fixierungseinrichtung gemäß Fig. 5 in Aufsicht nach Abnahme des Armauflagentischs, jedoch von der rechten Außenseite her;
- Fig. 9:: als abgewandeltes Bauteil der Auflage- und Fixierungseinrichtung gemäß Fig. 5 eine Gußschale mit Abstandsstiften für die Herstellung einer Gießform als Fixierungseinrichtung für die Hand als Untersuchungsobjekt;
- Fig. 10:: die mit der Gußform nach Fig. 9 hergestellte Gießform mit ausgeschnittenem Daumenbereich, aufgesetzt auf den Armauflagentisch gemäß Fig. 5, in Aufsicht;
- Fig. 11:: die Gießform als Fixierungseinrichtung gemäß Fig. 10 mit eingelegter und fixierter Hand;
- Fig. 12:: die Gußschale gemäß Fig. 9 in Aufsicht mit aufgelegtem vorderem Handabschnitt;
- Fig-13:: als weitere Abwandlung der Auflage- und Fixierungseinrichtung gemäß Fig. 1 und 5 eine Auflage- und Fixierungseinrichtung für ein menschliches Sprunggelenk mit darin fixiertem Fuß, in Aufsicht;
- Fig. 14:: die Auflageplatte der Auflage- und Fixierungseinrichtung gemäß Fig. 13 in Seitenansicht vor Ansatz der Körperextremität;
- Fig. 15:: als Abwandlung der in Fig. 1 und 5 dargestellten Ausführungsform die Untersuchungsvorrichtung mit einem Linearantrieb als Kraftquelle mit Linearachse, vorderem und hinterem Linearanschlag in der Frontalansicht mit abgenommener Gehäusewand;
- Fig. 16:: die Abwandlung gemäß Fig. 15 in der Seitenansicht mit Linearmotor, Linearachse, Linearanschlägen und Kraftumlenkung auf die Hauptgeräteachse;
- Fig. 17:: ein Zwillings-Reizelektrodenpaar zur Stimulation des untersuchten Nerven oder- Muskelbereichs;
- Fig. 18:: eine Halterungsvorrichtung für die Reizelektroden am Untersuchungsobjekt als Detailzeichnung;
- Fig. 19:: die Halterungsvorrichtung gemäß Fig. 18 mit Fixierungsband und Klemmver-schluß in Seitenansicht;
- Fig. 20:: als Detail aus Fig. 19 einen Abschnitt des Fixierungsbandes mit dem Klemmverschluß;
- Fig. 21:: eine abgewandelte Halterungsvorrichtung in Seitenansicht;
- Fig. 22:: als Detail aus Fig. 21 die breitere Halterlippe mit durchgezogenem Fixierungsband;
- Fig. 23:: als Detail der Halterungsvorrichtung gemäß Fig. 18 für die Reizelektroden ein Halterklötzchen für die Reizelektroden mit geänderter Höhe mit Halterlippe in Aufsicht;
- Fig. 24:: die Halterungsvorrichtung gemäß Fig. 23 in Vorderansicht;
- Fig. 25:: die Halterungsvorrichtung gemäß Fig. 23 in Seitenansicht;
- Fig. 26:: die Halterungsvorrichtung wie in Fig. 23 mit einem höheren Halterklötzchen;
- Fig. 27:: die Halterungsvorrichtung gemäß Fig. 26 in Vorderansicht;
- Fig. 28:: die Halterungsvorrichtung gemäß Fig. 26 in Seitenansicht;
- Fig. 29:: ein Sensorgehäuse mit Sensoren zur Aufnahme von elektrischen und akustischen Signalen bzw. Vibrationen in Ansicht von unten;
- Fig. 30:: das Sensorgehäuse mit Sensoren gemäß Fig. 29 in Seitenansicht;
- Fig. 31:: als Detail von Fig. 30 die Kugelgelenkhalterung für die Sensoreinheit;
- Fig. 32:: eine Abwandlung der Sensoreneinheit gemäß Fig. 29 mit Haftflächen und Vakuumanschlüssen in der Ansicht von unten;
- Fig. 33:: die Sensoreneinheit gemäß Fig. 32 in der Seitenansicht im Schnitt;
- Fig. 34:: die Sensoreneinheit gemäß Fig. 32 in der Aufsicht;
- Fig_ 35:: eine Multisensoreinheit mit Vorrichtung zur Darstellung, Speicherung und Verarbeitung von Meßwerten im Längsschnitt von einer Außenseite her;
- Fig. 36:: die Unterseite der Multisensoreinheit gemäß Fig. 35;
- Fig. 37:: die Multisensoremheit gemäß Fig. 35 in der Aufsicht von oben mit Bedien- und Anzeigefeldern.

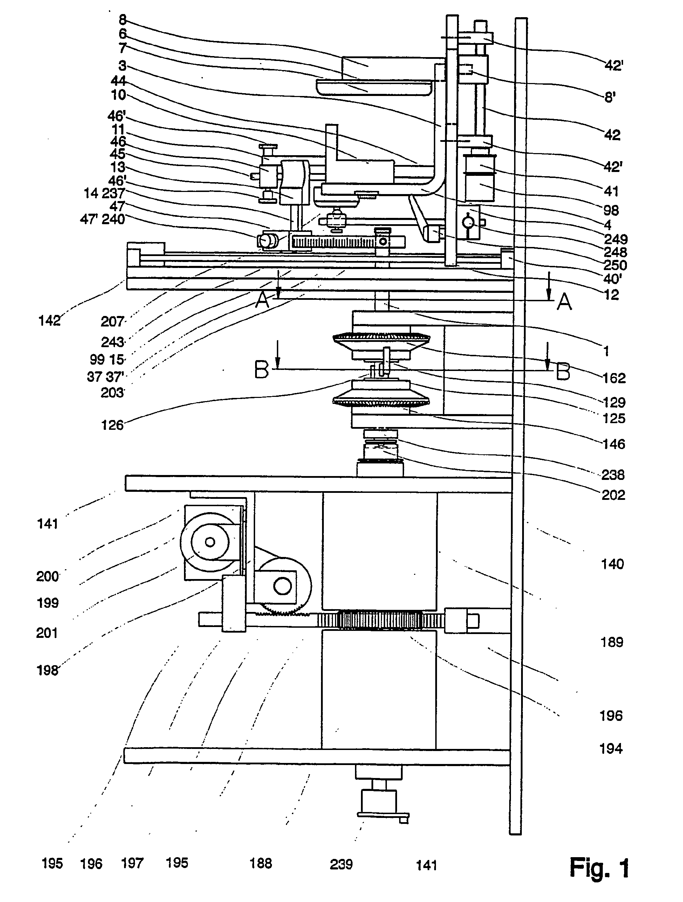

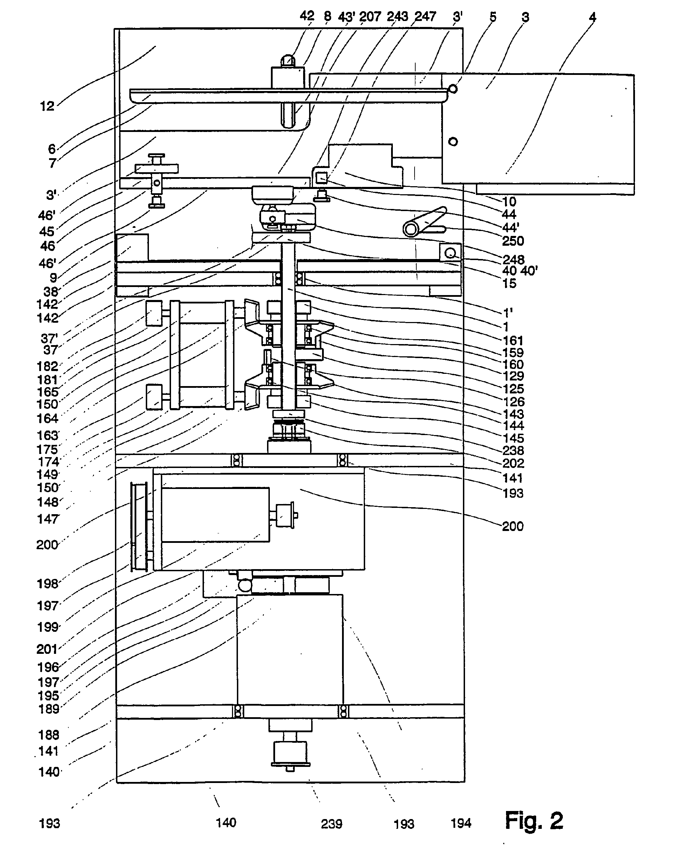

Bei der Ausführungsform der Vorrichtung gemäß Fig. 1 und 2 ist diese auf ihren (nur teilweise dargestellten) senkrechten Außenwänden 140 aufgestellt. In ihrem oberen Bereich ist an einer Gehäusequerwand 142, hier als Doppelwand ausgebildet, eine Auflage- und Fixierungseinrichtung 3, 3', 4, 6, 7, 9 für die zu untersuchende Hand nebst Handgelenk und anschließendem Unterarmabschnitt angeordnet. Dabei ist an der parallel zu einer Außenwand 140 im Abstand von dieser verlaufenden und senkrecht auf der Zwischenwand 142 angesetzten Verschienungsauflage 12 die Armauflage 3 angebracht, die sich in der gleichen Ebene in der Handauflage 3' fortsetzt. Im rechten Winkel zur Armauflage 3 und der Verschienungsauflage 12 ist die Handrückenstütze 6 mit Handrückenpolster 7 und dem Verschiebungssteg 8 angeordnet, die über den Verschiebungssteg 8 in einer angepaßten Ausnehmung 43' der Verschienungsauflage 12 vertikal verschiebbar sind.

Ferner schließt ebenfalls im rechten Winkel zur Armauflage 3 und zur Verschienungsauflage 12 die Armauflage 4 an. Zur Stütze und Fixierung der Unterseite des Handgelenks ist außerdem auf der Handauflage 3' die Handgelenkstütze 10 mittels der Träger- und Verschiebungsschiene 44 horizontal verschiebbar und durch eine Befestigungsschraube 44' arretierbar angesetzt. Zur Untersuchung des motorischen Systems an der Hand der untersuchten Person wird die Hand mit dem Handgelenk und dem anschließenden Unterarmabschnitt so in die Vorrichtung eingelegt, daß der Unterarm mit seiner Außenseite auf der Armauflage 3, das Handgelenk und die Schmalseite der Hand nebst kleinem Finger auf der Handauflage 3' aufliegen. Der Handrücken liegt dabei an der Handrückenstütze 6 mit Handrückenpolster 7 an, die Handfläche an der Handflächenstütze 9 und das Handgelenk mit seiner Unterseite an der Handgelenkstütze 10. Die Finger II -V werden zusätzlich an der Außenkante des Zeigefingers durch eine Fingerstütze 11 mit Verschiebeklötzchen 46 abgestützt.

Die Hand und das Handgelenk werden nach der Einführung in die Vorrichtung in der gewünschten bzw. erforderlichen Stellung fixiert, indem die Handrückenstütze 6 nebst Handrückenpolster 7 durch einen Elektromotor 41 mit Spindel 42, in die der Verschiebungssteg 8 mittels eines Verschiebeklötzchens 43 eingreift, vertikal verschoben werden, und indem anderseits die Handgelenkstütze 10 auf der Verschiebungsschiene 44 horizontal verschoben und in der dem Handgelenk angepassten Stellung mittels der Stellschraube 44' arretiert wird, wobei auch die Handgelenkoberseite abgestützt wird. Der Motor 41 und die Spindel 42 sind auf der der Armauflage 3 abgewandten Seite der Verschienungsauflage 12 mittels an dieser angesetzten Spindelführungen 42' angeordnet. Dem Motor 41 ist ein Strommesser 98 zugeordnet, der diesen abschaltet, sobald die Handrückenstütze 6 mit einer vorgegebenen Andruckskraft am Handrücken anliegt. Zusätzlich kann die Handrückenstütze 6 mit dem Verschiebungssteg 8 über ein Drehgelenk 8' im Bereich des Eingriffs in die Spindel um eine vertikale Achse schwenkbar sein. Die Fingerstütze 11 wird mittels des Verschiebeklötzchens 46 auf der Verschiebungsführung 45 an den Zeigefinger herangeführt und mit den Stellschrauben 46' arretiert.

Die Verschienungsauflage 12 mit den vorgenannten Bauteilen ist ihrerseits horizontal und parallel zu der Zwischenwand 142 auf einer oder mehreren Führungsschienen 40, die mit Halterungen 40' an der Zwischenwand 142 angebracht und als Spindeln ausgestaltet sind, mittels eines Elektromotors 38 verschieblich. Zur Untersuchung der Funktions- und Leistungsfähigkeit der Muskulatur eines Körperteils bzw. Nerv-/Muskel-Ausschnitts eines Körperteils - bei dem in Fig. 1 und 2 dargestellten Ausführungsbeispiel der Vorrichtung vorzugsweise die Daumenmuskulatur (Daumenadduktor und Daumenabduktor)- ist für den Daumen der eingeschobenen Hand die Daumenschale 13 mit Steg 14 vorhanden, der in den horizontal angeordneten Daumenhebel 15 eingreift und auf diesem mittels der Verschiebevorrichtung 47 und Arretierung 47' horizontal verschiebbar ist. In diese Daumenschale 13 wird der Daumen eingelegt. Der Daumenhebel 15 ist seinerseits an der vertikal angeordneten Hauptgeräteachse 1 angebracht, die ihrerseits durch die Zwischenwand 142 und eine weitere horizontale Zwischenwand 141 geführt ist und in Drehmagnete 188 und 189 als Kraftquelle eingreift. Am obersten Ende der Häuptgeräteachse 1 ist in dieser ein Laserpeilstrahler 37 mit Blende 37' eingesetzt, dessen Peilstrahl mit der Bewegungsachse des untersuchten Körperteils, wie zum Beispiel der Bewegungsachse von Daumenadduktor und Daumenabduktor, zur Deckung gebracht wird. In diesem Zustand werden die Hand nebst Handgelenk und Unterarm mittels der Armauflagen 3 und 4, der Handrückenstütze 6, der Handflächenstütze 9, der Handgelenkstütze 10 und der Fingerstütze 11 sowie der Verschienungsauflage 12 fixiert. Ferner wird der Daumen mittels der Daumenschale 13 und des Daumenhebels 15 mit der Hauptgeräteachse 1 der Vorrichtung fest verbunden. Über die Hauptgeräteachse 1 und den Daumenhebel 15 üben die Drehmagnete 188 und 189 als Kraftquelle auf die zu untersuchenden Muskeln definierbare Vor- und/oder Nachlasten aus.

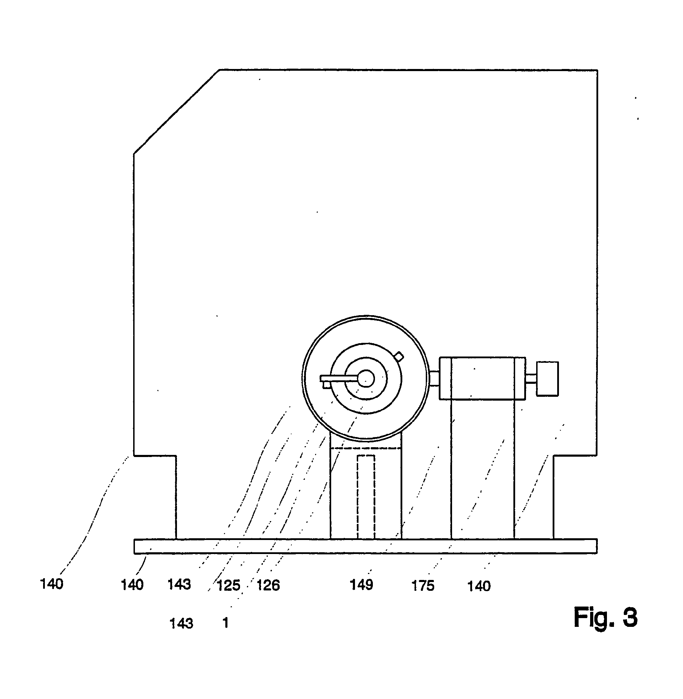

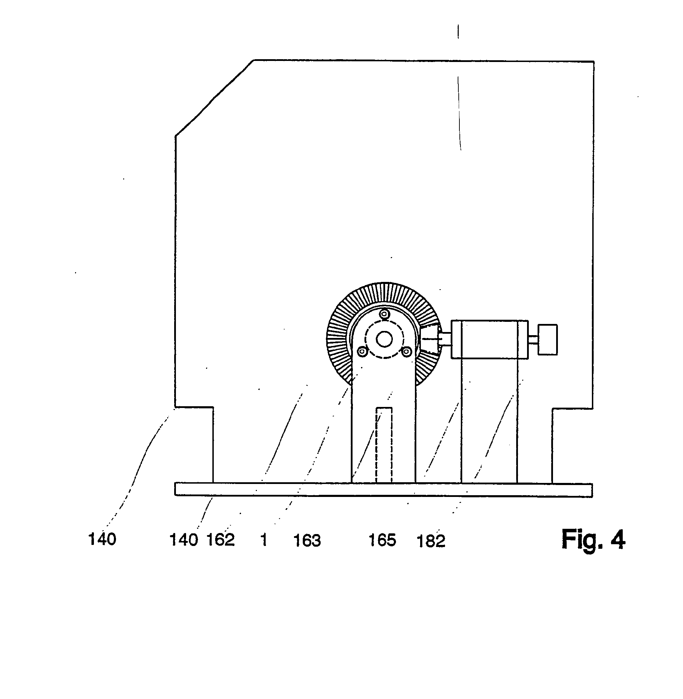

Wie in Fig. 1 und 2 ebenfalls sichtbar und in den Fig. 3 und 4 in Details dargestellt, hat die Hauptgeräteachse 1 unterhalb der Zwischenwand 142 einen mit ihr fest verbundenen Achsanschlag 125, der mit einem vorderen Rotationsanschlag 126 zusammenwirkt (Fig. 1 bis 3). Der Rotationsanschlag 126 ist auf einem Verstellrad 143 angebracht und kann über dieses im Winkel verstellt werden. Das Verstellrad 143 ist mittels eines Lagers 144 auf einem Führungsrohr 145 gelagert, das die Hauptgeräteachse 1 ohne Kraftschluß umgreift. Ferner ist das Verstellrad 143 auf der anschlagsfernen Seite mit einem Zahnkranz 146 versehen, in den ein Zahnrad 163 eingreift, das über eine Achse 164 von einem Verstellmotor 149 angetrieben wird, der seinerseits am Gehäuse 140 mittels einer oder mehrerer Halterungen 150 befestigt ist. An der Motorachse 164 ist ein Potentiometer 175 mittels der Potentiometerachse 174 angebracht, der die Stellung des vorderen Anschlags mißt. Der vordere Rotationsanschlag 126 verhindert Rotationsbewegungen des Daumenhebels 15 über diesen Anschlag hinaus nach vorne und damit Kontraktionen des Daumenadduktors, so daß eine isometrische Zuckung bzw. Anschlagszuckung bei einer definierten Muskellänge erzwungen wird.

Ferner wirkt der Achsanschlag 125 mit einem hinteren Rotationsanschlag 129 zusammen. Dieser ist auf einem Verstellrad 159 angebracht und kann über dieses ebenfalls im Winkel verstellt werden. Das Verstellrad 159 ist analog der Ausgestaltung beim vorderen Rotationsanschlag mittels eines Lagers 160 auf dem Führungsrohr 161 gelagert, das die Hauptgeräteachse 1 ohne Kraftschluß umgreift. Ferner ist das Verstellrad 159 auf der anschlagsfernen Seite mit dem Zahnkranz 162 versehen, in den ein Zahnrad 163' eingreift, das über eine Achse 164' von einem Verstellmotor 165 angetrieben wird, der seinerseits wieder am Gehäuse 140 mittels der Halterung 150 befestigt ist. Auch an der Motorachse 164' ist ein Potentiometer 182 mittels der Potentiometerachse 181 angebracht, der die Stellung des hinteren Anschlags mißt. Durch den hinteren Rotationsanschlag 129 werden Rotationsbewegungen des Daumenhebels 15 nach hinten verhindert und damit Dehnungen des Daumenadduktors, so daß Unterstützungszuckungen auf einer definierten Muskellänge ausgelöst werden können.

Die als Kraftquelle für die Belastung des Muskels eingesetzten Drehmagnete 188 und 189 sind hintereinander geschaltet und jeweils mittels Lagern 193 an der oberen bzw. unteren Zwischenwand 141 fest angeordnet. Ihre Innenteile sind mit der Hauptgeräteachse 1 verbunden und können um diese Innenteile rotieren. Zwischen den beiden Drehmagneten ist ein Zahnkranz 194 angeordnet, in den eine Zahnstange 195 eingreift, die in entsprechenden Zahnstangenlagern 196 geführt und linear bzw. horizontal beweglich ist. Diese Zahnstange steht ihrerseits über ein Zahnrad 197 und einen Übersetzungsriemen 198 mit einem Motor 199 in Verbindung, die an einer an der oberen Zwischenwand 141 angesetzten Halteplatte 200 befestigt sind. Der Motor 199 verstellt über die Zahnrad- bzw. Zahnstangenverbindung 194, 195, 197 die Drehmagnete 188, 189 derart, daß der Bewegungsbereich der untersuchten Extemität in den günstigen Arbeitsbereich oder Kennlinienbereich der Drehmagnete fällt. An der Achse des Motors 200 ist ein Potentiometer 201 angeordnet, der die jeweilige Stellung der Drehmagnete 188, 189 mißt.

Zur Verhinderung einer zu hohen, auf die untersuchte Extremität einwirkenden und damit schädlichen Kraft ist auf der Hauptgeräteachse 1 im Anschluß an die Drehmagnete 188, 189 eine elektronisch ansteuerbare und in ihrem Arbeitsbereich einstellbare Rutschkupplung 202 angesetzt. Statt dieser Rutschkupplung oder zusätzlich zu ihr kann in vereinfachter Form die Verbindung des Daumenhebels 15 mit der Hauptgeräteachse 1, wie aus Fig. 1 ersichtlich, durch einen Bolzen 203 mit kraftdefinierter Sollbruchstelle erfolgen, der bei Überschreitung eines definierten Grenzwerts der Krafteinwirkung der Kraftquelle 188, 189 bricht und damit eine weitere Krafteinwirkung auf den eingespannten Daumen der Testperson ausschließt.

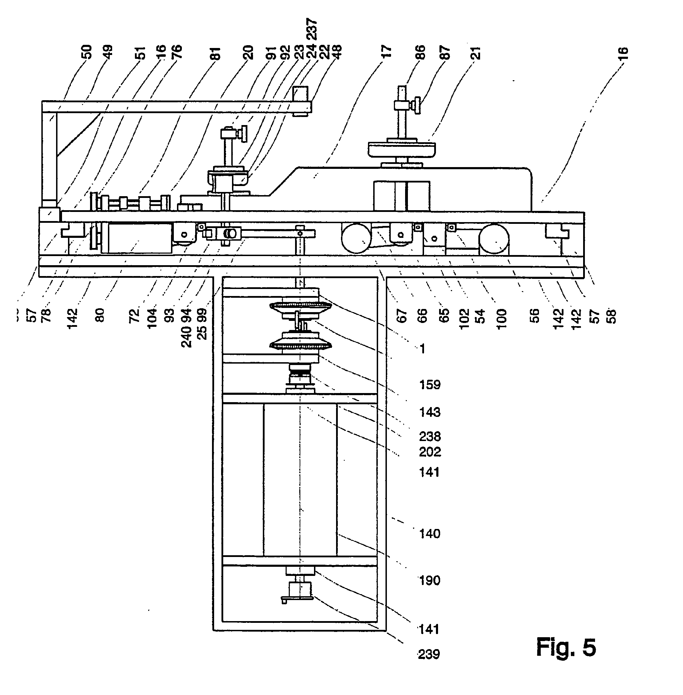

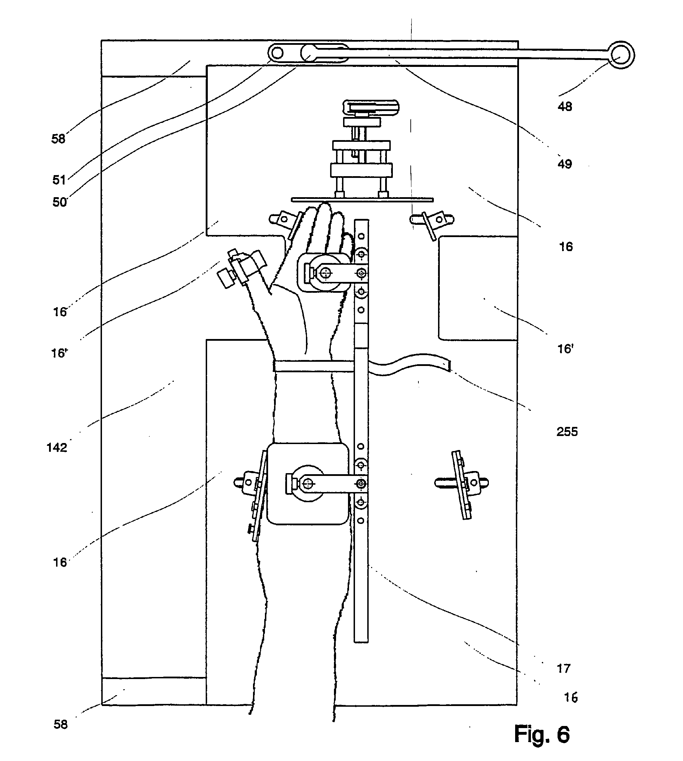

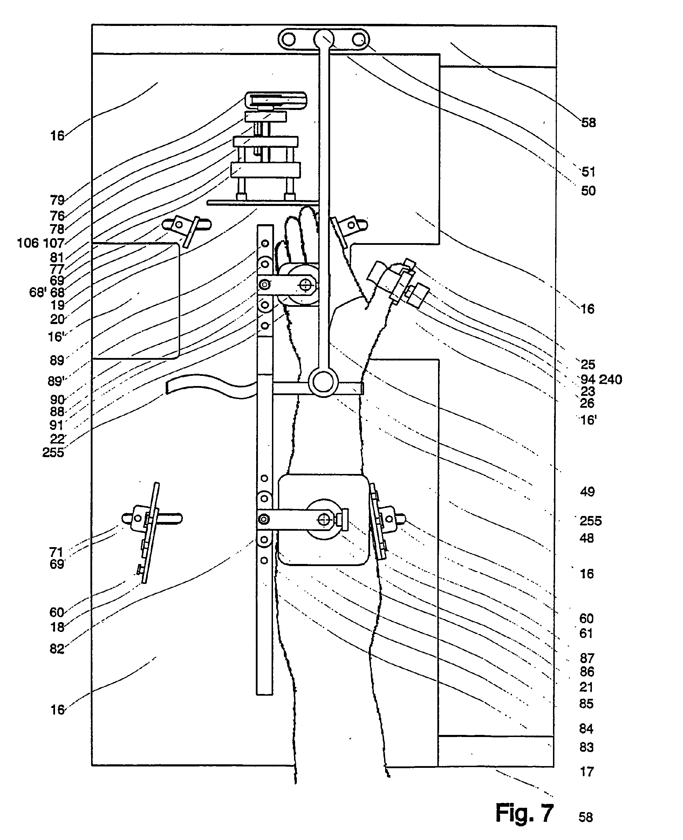

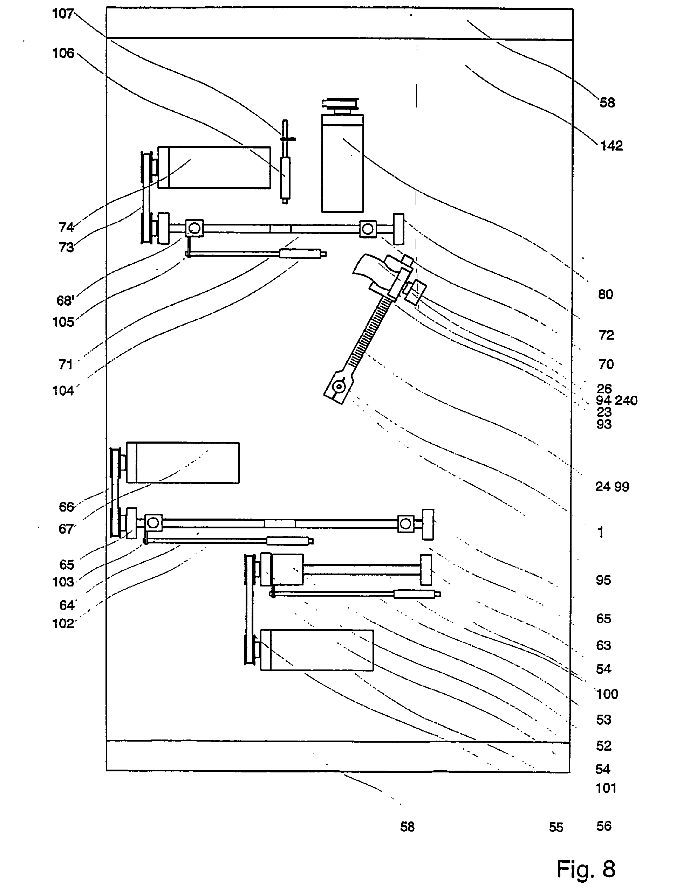

Eine andere Ausführungsform der erfindungsgemäßen Vorrichtung ist in Fig. 5 bis 8 dargestellt. Auch diese Ausführungsform ist auf ihren (nur teilweise dargestellten) senkrechten Außenwänden 140 aufgestellt. Hier ist auf der Gehäusequerwand 142 im vertikalen Abstand über dieser in Führungen bzw. Auflagen 58 an wenigstens zwei einander gegenüberliegenden Außenseiten der Gehäusequerwand 142 ein Armauflagentisch 16 horizontal verschieblich angeordnet, auf den Arm und Hand aufgelegt und zur Untersuchung fixiert werden. Im Bereich des Daumens hat der Armauflagentisch 16 einen Ausschnitt 16', in dessen Bereich die Daumenschale 23 mit Steg 24 von dem unterhalb des Armauflagentischs 16 angeordneten Daumenhebel 25 (entsprechend der Daumenschale 13 mit Steg 14 und Daumenhebel 15) senkrecht nach oben ragt zur Einlage des Daumens der eingesetzten Hand. Der Daumenhebel 25 ist seinerseits wie bei der Ausführungsform gemäß Fig. 1 und 2 an der vertikal angeordneten Hauptgeräteachse 1 angebracht, die ihrerseits durch die doppelte Gehäusequerwand 142 und die weitere horizontale Zwischenwand 141 geführt ist und in einen Elektromotor, vorzugsweise Hallmotor 190 als alternative Kraftquelle zu den Drehmagneten 188 und 189 eingreift.

Auf dem Armauflagentisch 16 ist mittig und in Längsrichtung des aufgelegten Arms nebst Hand die Armanlage 17 angebracht, an der die Arminnenseite und die Handkante angelegt werden, und zwar je nach der untersuchten rechten oder linken Extremität und je nachdem, ob die Handfläche nach oben oder unten gerichtet ist, jeweils links oder rechts an der Armanlage.

Der Armauflagentisch 16 wird in den Führungen 58 durch einen Motor 56, der unterhalb des Armauflagentischs auf der Gehäusequerwand 142 montiert ist, über ein Verschiebeklötzchen 52, das fest an der Unterseite des Armauflagentischs 16 angesetzt ist und in einer Spindel 53 geführt wird, mittels eines Übersetzungsriemes 55 verschoben, um den aufgelegten Arm seitwärts in die richtige Position zu bewegen. Die Spindel 53 ist ihrerseits in Führungen 54 an der Unterseite des Armauflagentischs 16 gelagert. Die Position des Armauflagentischs 16 kann über einen Linearmesser 100, der über das Verbindungsstück 101 mit dem Verschiebungsklötzchen 52 verbunden ist, gemessen und für spätere Untersuchungen wiederholbar festgelegt werden.

Auch hier wird die Bewegungsachse des untersuchten Körperteils mittels des Laserpeilstrahlers 37 mit dessen Peilstrahl zur Deckung gebracht, um den zu untersuchenden Körperteil in die richtige Position zu bringen. Alternativ oder zusätzlich zum Laserpeilstrahler 37 kann, wie in Fig. 5 bis 7 dargestellt, der Laserpeilstrahler 48 an einem Schwenkarm 49 angeordnet sein, der an einer Außenseite der Zwischenwand 142 auf einer der Führungen 58 drehbar eingesetzt ist und über den Armauflagentisch und die aufgelegte Extremität geschwenkt wird. Sodann wird die Bewegungsachse des untersuchten Körperteils mit dem vom Laserpeilstrahler 48 senkrecht nach unten gerichteten Peilstrahl zur Deckung gebracht, wobei der Peilstrahl genau mit der Hauptgeräteachse 1 übereinstimmt. In diesem Zustand werden der Arm mit dem Armklemmpolster 21 und dem Handflächenklemmpolster 22 fixiert, wobei das Armklemmpolster 21 durch den Haltebügel 85 und das Handflächenklemmpolster 22 durch den Haltebügel 90 an der Oberseite der Armanlage mittels der Bügelbefestigungen 82 und 88 angesetzt sind. Dabei erfolgt die Befestigung in an sich bekannter Weise mittels Schrauben oder Bolzen 84 und 89' in Bohrungen 83, 89. Die Haltebügel 85 und 90 sind jeweils in Längsrichtung der Armanlage versetzbar zur Anpassung an die jeweiligen anatomischen Gegebenheiten der Versuchsperson, indem jeweils Lochreihen 83, 89 in der Armanlage 17 vorgesehen werden. Das Armklemmpolster 21 und das Handflächenklemmpolster 22 können dabei jeweils um 180° gedreht in der Armanlage 17 eingesetzt und befestigt werden, je nachdem welche Extremität untersucht wird. Zur Anpassung des Armklemmpolsters und des Handflächenklemmpolsters an die jeweilige Höhe der Handfläche und des Unterarms im aufgelegten Zustand haben das Armklemmpolster den Verschiebungsstab 86 mit Arretierung 87 und das Handftächenktemmpolster 22 den Verschiebungstab 91 mit Arretierung 92, die jeweils senkrecht auf dem Armauflagentisch 16 befestigt sind und senkrecht aufragen sowie in ihrer Höhe die Armanlage 17 überragen.

Die der Handkante gegenüberliegende Außenseite der Finger II bis V wird über eine, ebenfalls auf den Armauflagentisch 16 verschieblich angeordnete, vorzugsweise gepolsterte Fingerstütze 19 abgestützt, die das Drehgelenk 68 mit Drehgelenkstift 68' zur Anpassung an die jeweilige Fingerform hat. Entsprechende Fingerstützen sind beidseits der Armanlage 17 angebracht zur Abstützung der Finger der rechten oder der linken Hand.

Der Drehgelenkstift 68' ist durch ein Langloch 69 im Armauflagentisch 16 auf dessen Unterseite mit einem Verschiebeklötzchen 70 verbunden, das durch eine Spindel 71 mit gegenläufigen Gewinden und einen Motor 74 die Fingerstütze 19 zur Verschienung an die eingesetzte Hand führt, wobei sich gleichzeitig die zweite Fingerstütze gegenläufig zur Armanlage 17 bewegt. Die Spindel 71 ist mit Spindelführungen 72 ebenso an der Unterseite des Armauflagentischs 16 befestigt wie der Motor 74, der die Spindel über einen Übersetzungsriemen 73 antreibt.

Entsprechend den Fingerstützen 19 sind im Bereich des Armklemmpolsters 21 beidseits der Armanlage 17 Armstützen 18 für die jeweilige Außenseite des Arms vorgesehen, die in ihrer technischen Ausgestaltung mit dem Drehgelenk 61, dem Drehgelenkstift 61', dem Verschiebeklötzchen 63 und der Spindel 64 im wesentlichen entsprechen, wobei die Armstütze wiederum über einen Motor 67 mit Übersetzungsriemen 66 und die Spindel an den aufgelegten Arm herangeführt wird.

Die Position der Fingerstütze 19 kann über einen Linearmesser 104, der über ein Verbindungsstück 105 mit dem Verschiebungsklötzchen 70 verbunden ist gemessen und derart für spätere Untersuchungen festgelegt werden. Ebenso kann die Position der Armstütze 18 über einen Linearmesser 102, der über das Verbindungsstück 103 mit dem Verschiebungsklötzchen 63 verbunden ist, gemessen und für spätere Untersuchungen festgelegt werden.

Zur Abstützung der Fingerspitzen ist bei dem Ausführungsbeispiel gemäß Fig. 5 bis 8 eine auf dem Armauflagentisch 16 verschiebliche Stütze 20 angeordnet, die wie bei der Fingerstütze 19 mit einem unterhalb des Armauflagentischs in einer Spindel 76 geführten Verschiebeklötzchen 75 fest verbunden ist. Die Spindel ist in den Führungen 77 am Armauflagentisch gelagert und wird durch den Motor 80 und den Übersetzungsriemen 78 bewegt, wobei durch das Verschiebeklötzchen 75 die Stütze 20 mit ihren Teleskopführungen 81 an die Fingerspitzen herangeführt und positioniert werden. Auch die Position dieser Fingerspitzenstütze 20 kann über einen Linearmesser 106 der über ein Verbindungsstück 107 mit dem Verschiebungsklötzchen 75 verbunden ist gemessen und für spätere Untersuchungen festgelegt werden.

Der Daumen der so in der Vorrichtung eingesetzten und fixierten Extremität wird von hinten durch die Daumenschale 23, im Gegensatz zur Daumenschale 13 beim Ausführungsbeispiel gemäß Fig. 1 und 2, gestützt und mit einem Halteband 26 fixiert. Der Daumensteg 24 wird auch bei diesem Ausführungsbeispiel in Anpassung an die Größe von Hand und Daumen der untersuchten Extremität auf dem Daumenhebel 25 horizontal verschoben und mittels der Arretierung 94 fixiert.

Auch bei dieser Ausführungsform hat die Hauptgeräteachse 1 unterhalb der Zwischenwand 142 den mit ihr fest verbundenen Achsanschlag 125 und den vorderen Rotationsanschlag 126 und den hinteren Rotationsanschlag 129 und deren zusätzliche Bauteile wie bei der Ausführungsform gemäß Fig. 1 bis 4. Die vorliegende Ausführungsform kann auch statt des oben erwähnten Hallmotors die Drehmagnete 188 und 189 als Kraftquelle haben.

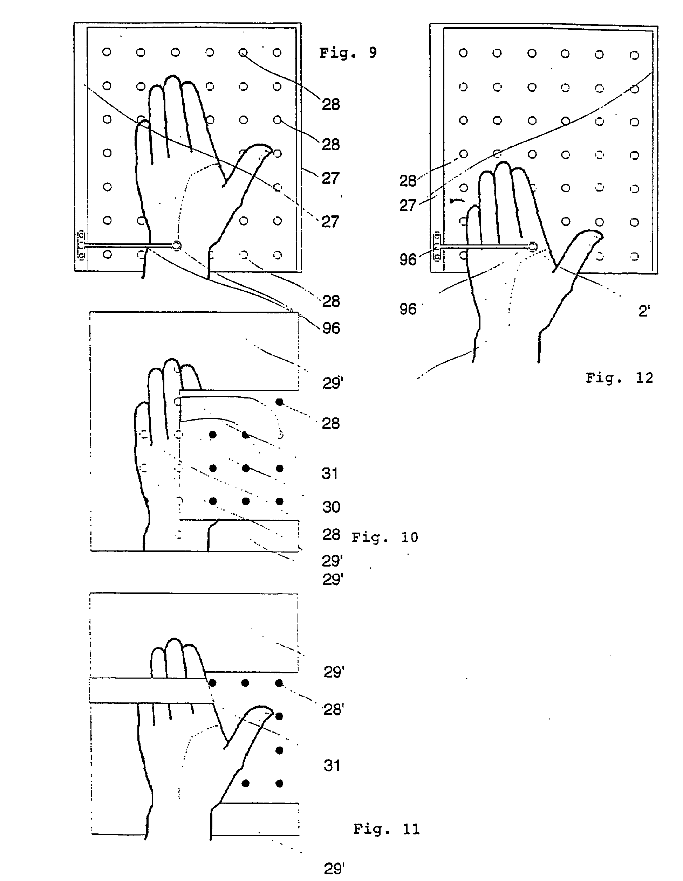

In Fig. 9 bis 12 ist als Bestandteil einer weiteren vorteilhaften Ausführungsform der erfindungsgemäßen Vorrichtung eine andere Auflage- und Fixierungseinrichtung für die zu untersuchende Hand dargestellt, die bei der Ausführungsform gemäß Fig. 5 bis 8 an die Stelle der Auflage- und Fixierungseinrichtung für die Hand und den Arm der Armanlage 17 mit Armstütze 18, Fingerstütze 19 und Fingerspitzenstütze 20 sowie deren beschriebene Vorrichtungen und Ausgestaltung tritt. Bei dieser wird der den Gegenstand der Untersuchung dienende Teil der Extremität der Versuchsperson, im dargestellten Ausführungsbeispiel die Hand, in eine Gußschale 27 auf Abstandsstifte 28 aufgelegt, die in den Boden der Gußschale 27 lösbar eingesteckt sind. Den dafür vorgesehenen Bohrungen im Boden der Gußschale 27 entsprechen Bohrungen 28' im Armauflagentisch 16. An einem der oberen Außenränder der Gußschale 27 ist der dem Schwenkarm 49, 50 mit Laserpeilstrahler 48 entsprechende Schenkarm mit Laserpeilung 96 derart angeordnet, daß bei Aufsatz der Gußform auf dem Armauflagentisch 16 der Lichtpeilstrahl genau auf die Haupt-geräteachse 1 der Vorrichtung gerichtet ist, wenn die Bohrungen im Boden der Gußschale 27 und im Armauflagentisch sich decken. Nach Auflage der Hand oder den Handabschnitts auf den Abstandsstiften 28 der Gußschale 27 wird der schwenkbare Laserpeilstrahler 96 über die Extremität geschwenkt und der Peilstrahl mit der Bewegungsachse der Testperson zur Deckung kommt. In dieser Position wird die Extremität mit einem geeigneten Kunststoff oder sonstigen Gießmaterial so eingegossen, daß die Form des Handrückens und der Finger sowie gegebenenfalls des Handgelenks abgenommen wird und ein individuelles Handbett entsteht. Dabei werden die Abstandsstifte 28 mit eingegossen, aus der so entstandenen Gußform 29 wird nach Herausnahme aus der Gußschale ein Daumenausschnitt 30 herausgeschnitten. Diese Gußform wird mittels der eingegossenen, mit ihren unteren Enden aus ihr hervorstehenden Abstandsstiften 28 in die erwähnten Bohrungen des Armauflagentischs 16 eingesetzt. Zu Untersuchung und Fixierung legt die Versuchsperson, auch im Wiederholungsfall, die Hand oder den Handabschnitt lediglich in die Form ein. Zur zusätzlichen Fixierung in der Form kann ein an der Gußform 29 angebrachtes Fixierungsband 31 vorgesehen sein. Im übrigen entspricht die Vorrichtung der Ausführungsform gemäß Fig. 5 bis 8, insbesondere auch hinsichtlich der Fixierung des Daumens mit der Daumenschale 23 und ihrer Verbindung über den Daumensteg 24 und den Daumenhebel 25 mit der Hauptgeräteachse 1.

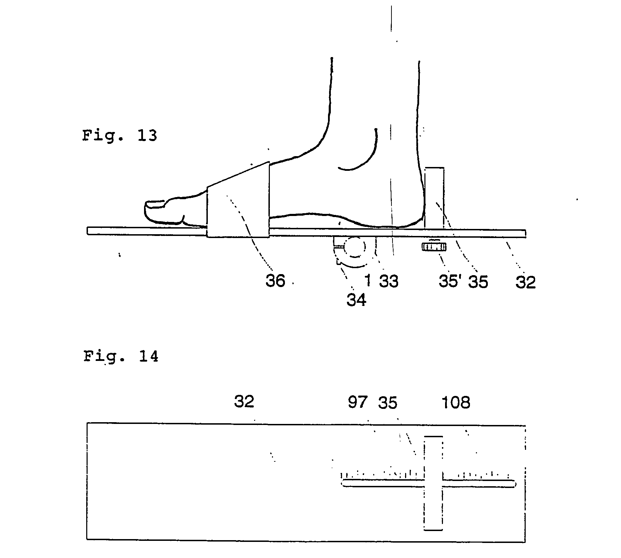

In Fig. 13 und 14 ist als Variante eine Auflage- und Fixierungseinrichtung zur Untersuchung von Muskeln dargestellt, die über große Gelenke wirken, wie beispielsweise das obere Sprunggelenk, das Ell-bogengelenk oder das Kniegelenk. Diese Ausführungsform besteht aus einer Auflageplatte 32 für das hier dargestellte Sprunggelenk, die auf ihrer Unterseite einen Haltering 33 hat, mit dem sie an der Hauptgeräteachse 1 durch Aufstecken und Verschraubung befestigt wird. Die Auflageplatte 32 hat zumindest über einen Teil ihrer Länge ein Langloch 96, in dem ein auf der Oberseite der Auflageplatte angeordneter Stützklotz 35 mit einem Stift greift und mittels Verschraubung 35' arretierbar ist. Der Stützklotz 35 wird nach dem Aufsetzen des Fußes der Versuchsperson so arretiert, daß die Bewegungsachse des zu untersuchenden Körperteils (hier des Sprunggelenks) über der Hauptgeräteachse 1 der Vorrichtung zu liegen kommt.

Zusätzlich wird die Extremität durch ein Fixierungsband 36 an der Auflageplatte 32 befestigt. Eine Meßskala 108 am Langloch 97 ermöglicht die Messung der Position des untersuchten Körperteils und damit die Festlegung für wiederholte Untersuchungen desselben Objekts.

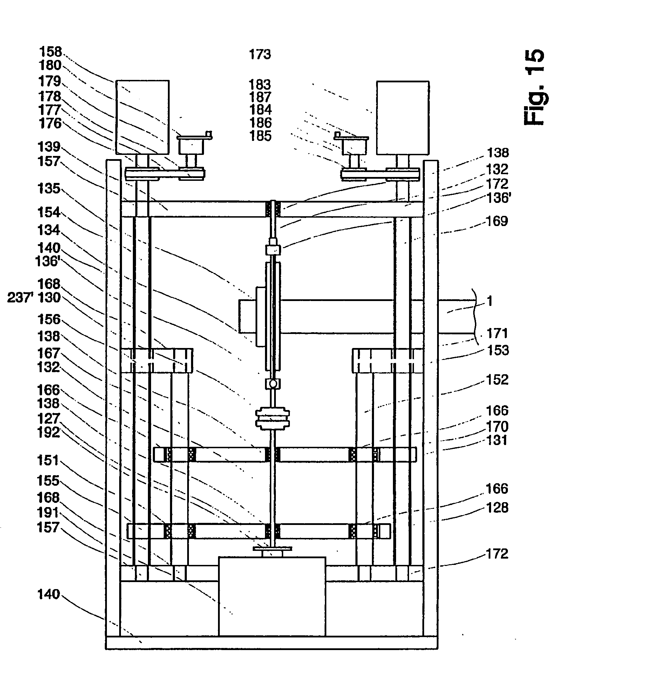

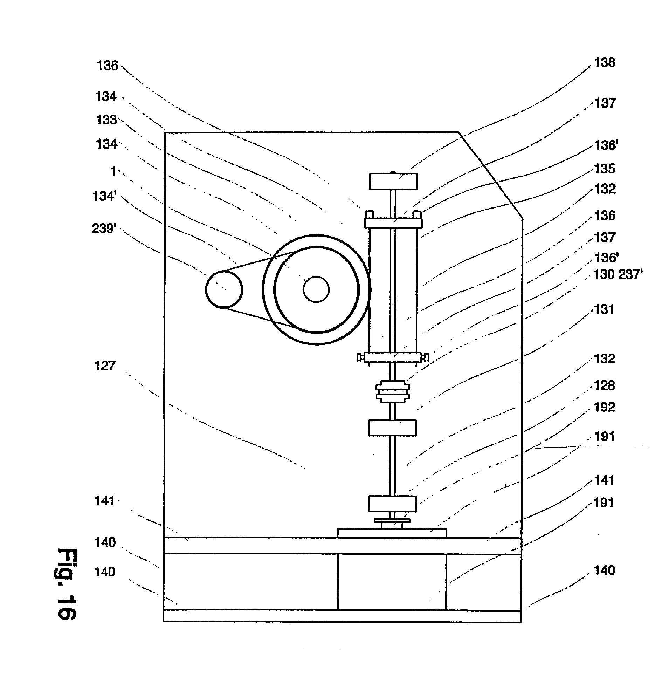

In den Fig. 15 und 16 ist eine weitere Ausführungsform der Kraftquelle und der Vorrichtungen zur Begrenzung der Kontraktion und der Dehnung des untersuchten Muskels wie z. B. des Daumenadduktors dargestellt, die an die Stelle der in den Fig. 1 bis 4 bzw. in Fig. 5 dargestellten Ausführungsformen dieser Teile der erfindungsgemäßen Vorrichtung treten. Hingegen entsprechen die Auflage- und Fixierungseinrichtungen für die zu untersuchenden Körperteile den vorstehend beschriebenen und schließen sich in der Darstellung gemäß Fig. 15 an die obere Zwischenwand des Gehäuses mit dem sichtbaren Ausgang der Hauptgeräteachse 1 an. Diese Ausführungsform hat als Kraftquelle einen Linearantrieb 191 in Form eines Linearmotors oder Hubmagneten, der über die Linearachse 132 und eine Übersetzung bzw. Kraftumlenkung 133 bis 137 Kraft auf die rotierend bewegliche Hauptgeräteachse 1 überträgt. Dabei sitzt auf der Hauptgeräteachse 1 im Bereich ihres Zusammentreffens mit der Linearachse 132 ein Übersetzungsrad 133, ein Übersetzungsseil 134, dessen eine Hälfte oben über das Übersetzungsrad 133 zu einer unteren Seilklemmung 136 auf der Linearachse 132 läuft und fixiert wird und dessen andere Hälfte unten über das Übersetzungsrad 133 zu einer oberen Seilklemmung 136 auf der Linearachse 132 läuft und fixiert wird. Auf der dem Übersetzungsseil 134 gegenüberliegenden Seite der Linearachse 132 ist ein Gegenzugseil 135 in Klemmungen 136', die ihrerseits über Klemmungsstege 137 an der Linearachse 132 befestigt sind, angebracht, das die einseitige Belastung und Durchbiegung der Linearachse 132 verhindert.

Die Linearachse 132 ist mit ihrem dem Motor bzw. Hubmagneten 191 gegenüberliegenden Ende im Bereich der Gehäuseaußenseite in einem Führungssteg 139 mit Gleitlager 138 gelagert, der seinerseits an der Außenwand 140 bzw. Zwischenwand 141 des Gehäuses angesetzt ist. Mit der Linearachse 132 ist ein vorderer Achsanschlag 127 im Anschluß an den Linearantrieb fest verbunden, der als Anschlag für den quer zur Linearachse 132 angeordneten Linearanschlag 128 dient. Dieser Linearanschlag 128 besteht aus einem Steg, durch den die Linearachse 132 in einem Gleitlager 138 hindurchführt. Er wird durch senkrecht zu ihm stehende Führungsstangen 152, 167 geführt und ist an diesen über Gleitlager 151, 166 parallel zur Linearachse verschieblich. Die Führungsstangen 152, 167 sind mittels Haltestegen 153, 168 fest mit der Gehäusewand 140 bzw. Zwischenwand 141 verbunden. Die Verschiebung erfolgt durch eine Spindel 154, die in einem Gewinde 155 durch den Linearanschlag 128 hindurchläuft. Der Linearanschlag 128 wird über die Spindel 154 mittels des Motors 158 bewegt. Durch diese Ausgestaltung mit dem vorderen Linearanschlag 128 am Achsanschlag 127 sind Bewegungen der Hauptgeräteachse 1 im Uhrzeigersinn und damit beispielsweise Kontraktionen des Daumenadduktors - begrenzt und wird eine isometrische bzw. Änschlags-Zuckung bei einer definierten Muskellänge erzwungen.

Ferner hat die in Fig. 15 und 16 dargestellte Ausführungsform einen mit der Linearachse 132 fest verbundenen hinteren Achsanschlag 130, gegen den ein hinterer Linearanschlag 131 anschlägt. Dieser hintere Linearanschlag 131 ist ebenfalls in den Führungsstangen 152, 167 geführt. Er wird über die Spindel 169 mit Motor 173 bewegt, deren Ausgestaltung der beschriebenen Bewegung des vorderen Linearanschlags 128 entspricht.

Zur Messung der jeweiligen Stellung der Linearachse 132 ist über das Übersetzungsrad 133 ein Potentiometer 239' angeschlossen. Ferner ist auf der Linearachse 132 ein Kraftmesser 237' zur Messung von Zug und Druck angeordnet. An den Spindeln 154 und 169 ist jeweils ein Potentiometer 180 bzw. 187 zur Messung der Position des vorderen Linearanschlags 128 bzw. des hinteren Linearanschlags 131 angebracht, In der dargestellten Ausführungsform geschieht dies über Übersetiungsräder 176 und 178 mit Übersetzungsseil 177 bzw. über Übersetzungsräder 183 und 185 mit Übersetzungsseil 184.

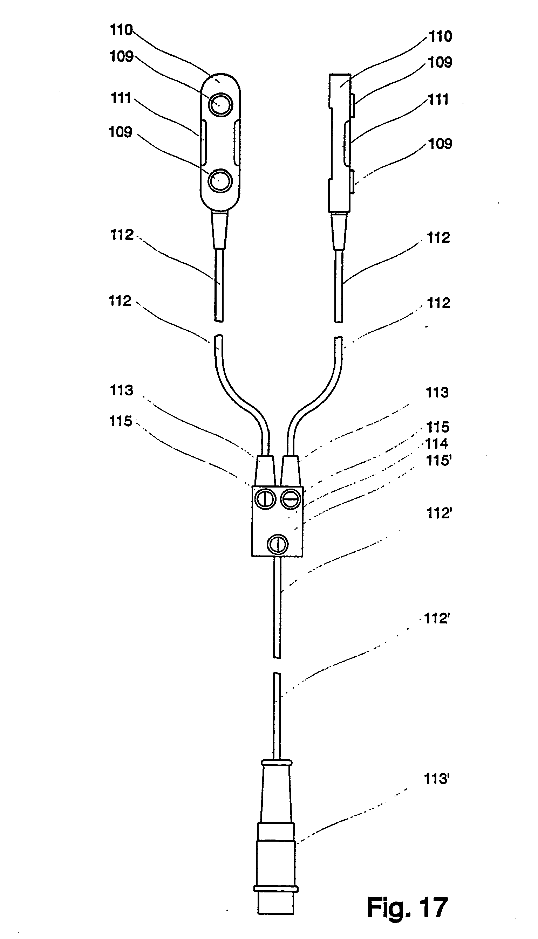

Die Stimulation des motorischen Systems zu seiner Untersuchung und insbesondere zur Untersuchung der Funktions- und Leistungsfähigkeit der Muskulatur erfolgt in dem zu untersuchenden Bereich wie zum Beispiel der Hand der Testperson durch Reizgeber zur Kontraktionsauslösung. Diese kann als willkürliche Auslösung, zentrale oder periphere Magnetstimulation, Reflexstimulation durch schnelle Lastveränderungen bzw. Muskellängenveränderungen oder auch durch periphere elektrische Stimulation erfolgen. Dabei ermöglichen die schnellen Last- bzw. Muskellängenveränderungen durch die Kraftquelle unter den Bedingungen der hier dargestellten Fixierungseinheit und der Anschläge erstmalig einstellbare und wiederholbare Reflexausauslösungen. In Fig. 17 ist als vorteilhafte Ausführungsform zur elektrischen Stimulation ein Zwillings-Reizelektrodenpaar 109, 110 dargestellt, bei dem jeweils in der Platte 110 als Halterung zwei Reizelektroden 109 eingesetzt sind, die über Kabel und Stecker 112, 113 an ein Schaltkästchen 114 angeschlossen sind. Dieses Schaltkästchen 114 weist für jedes der Reizelektrodenpaare 109/110 Einzelschalter 115 sowie einen Gesamtschalter 115' auf. Mit diesen Schaltern können wahlweise eine oder beide der Reizelektrodenpaare freigeschaltet oder abgeschaltet werden. Ferner stehen die Reizelektrodenpaare über das Kabel 112' und den Steckeranschluß 113' mit der in Fig. 17 nicht dargestellten Steuerungselektronik der Vorrichtung in Verbindung.

Statt der dargestellten Zwillings-Elektrodenpaare 109, 110 kann im Sinne der Erfindung auch nur ein solches Elektrodenpaar gewählt werden. Ebenso kann, wenn auch weniger vorteilhaft, statt eines Reizelektrodenpaars 109 nur eine Reizelektrode allein oder als Zwillingselektroden 109, 110 verwandt werden.

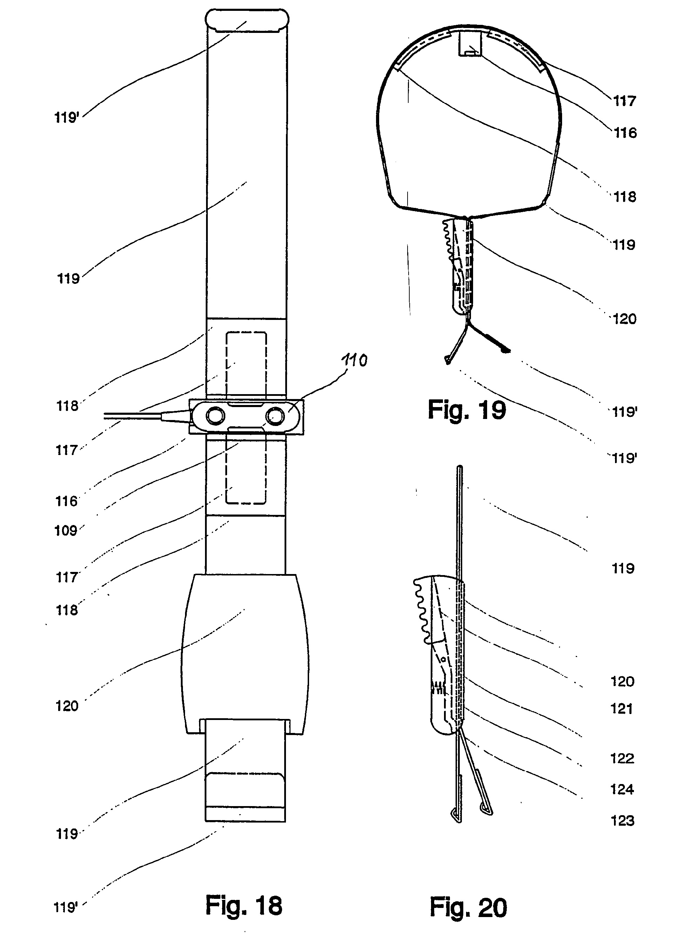

In den Fig. 18 bis 22 sind verschiedene Halterungsvorrichtungen für die verkippungs- und verschiebungssichere Anbringung der Reizelektroden, insbesondere von Reizelektroden 109, 110 an der für die Stimulation des motorischen Systems der Testperson erforderlichen Stelle des Körpers dargestellt. Wie aus Fig. 18 ersichtlich, wird das Reizelektrodenpaar 109, 110 in ein Halterklötzchen 116 eingesetzt, das an seiner den Reizelektroden und der Oberfläche des untersuchten Körperteils abgewandten Seite eine elastische, jedoch weitgehend starre Halterlippe 117 hat, die beidseits über das Halterklötzchen 116 hinaus reicht. Mit dieser Halterlippe 117 ist es in Haltetaschen 118 eines weich-elastischen Fixierungsbandes 119 eingesetzt. Das Fixierungs-band mit den so an ihm befestigten Reizelektroden 109 wird an der für die Stimulation erforderlichen Körperstelle wie zum Beispiel am Unterarm angelegt und mittels eines Klemmverschlusses 120 stufenlos anpassbar und lösbar angesetzt.

Der Klemmverschluß 120 hat, wie in Fig. 19 bis 21 sichtbar, den Verschlußöffner 121 mit der Klemmungsachse 122 und der Klemmungsfeder 124. Bei der Betätigung des Verschlußöffners 121 öffnet sich die Klemmung 123, so daß das Fixierungsband 119 durch den Verschluß hindurchgeführt, in seiner Länge verstellt oder aus dem Verschluß gänzlich herausgezogen werden kann. Nach der Anlegung und Anpassung des Fixierungsbands mit dem Reizelektrodenpaar 109 werden diese durch den Verschluß 120 in der vorgesehenen Andruckspannung gehalten. Eine Lösung ohne Betätigung des Verschlußöffners 121 ist nicht möglich, jedoch umgekehrt eine Nachspannüng des Fixierungsbandes 119.

Zweckmäßigerweise hat das Fixierungsband 119 an seinen beiden Enden Verstärkungen bzw. Versteifungen 119' zur besseren Einfädelung der Enden des Fixierungsbandes 119 in den Verschluß 120.

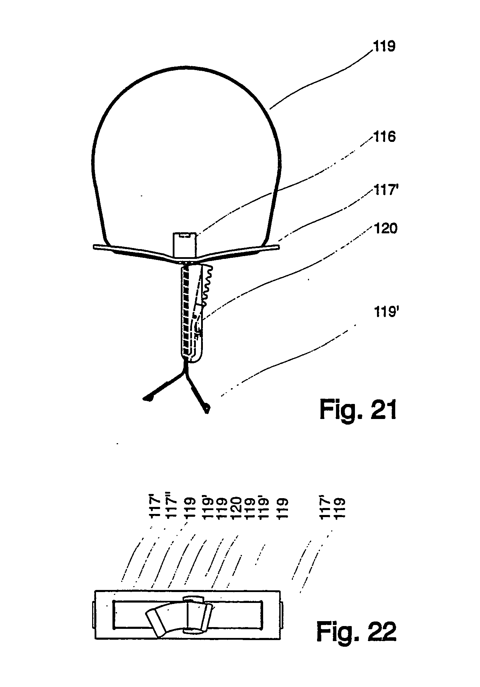

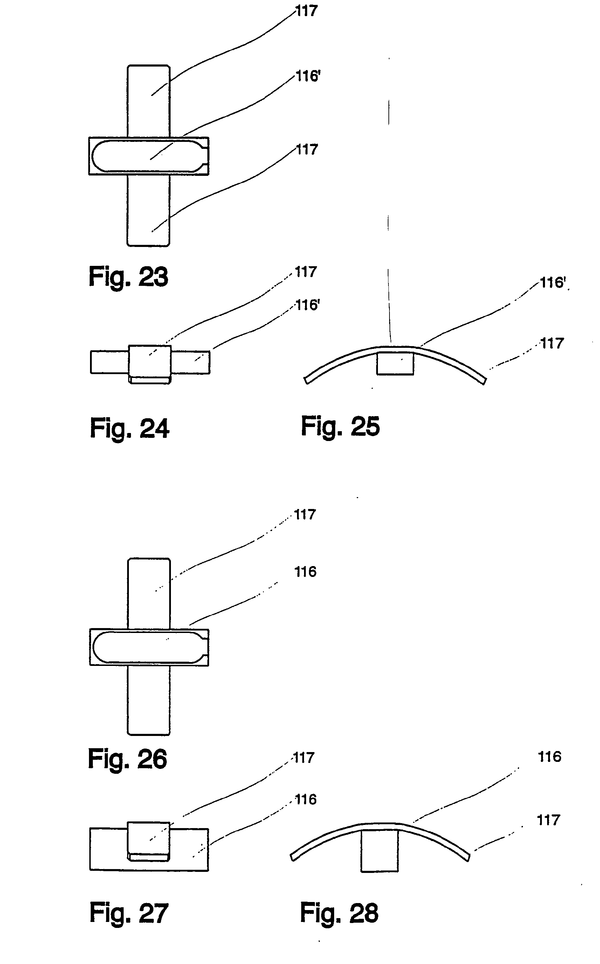

In den Fig. 21 und 22 ist eine abgewandelte Halterungvorrichtung für das Reizelektrodenpaar 109, 110 gezeigt. Dabei weist das Fixierungsband 119 nicht die Fixierungstaschen 118 auf; stattdessen ist die Halterlippe 117' breiter als das Fixierungsband 119 und weist sie an ihren beiden Längsenden Schlitze 117" auf, die der Breite des Fixierungsbands 119 entsprechen und durch die dieses gezogen und mit seinen beiden Enden in den Verschluß 120 eingeführt wird. Der Verschluß 120 kann dabei an frei wählbarer Stelle des Fixierungsbands 119 angeordnet sein.

In den Fig. 23 bis 25 sowie 26 bis 28 sind Halterklötzchen 116, 116' in unterschiedlicher Höhe für die Reizelektroden 109, 110 dargestellt, die jeweils austauschbar an der Halterungsvorrichtung angebracht und jeweils nach der für die Stimulation vorgesehenen Körperstelle ausgewählt werden können. Insbesondere kann damit der Druck auf die Reizelektrodenplatte 110 und damit die Reizelektroden unterschiedlich groß gehalten und können diese unterschiedlich tief in das Gewebe der untersuchten Extremität eingedrückt werden.

Die Ermittlung der Messwerte bei der jeweiligen Untersuchung, die die Grundlage der elektronischen oder sonstigen Auswertung bilden, erfolgt entweder durch Sensoren mechanischer Funktionen, Kraftmesser, Beschleunigungsmesser, Potentiometer oder durch Sensoren für thermische und elektrische Funktionen. Die mechanischen Sensoren mit Ausnahme des Vibrationsmessers sind in die erfindungsgemäße Vorrichtung als Bauteile integriert und aus den Darstellungen der Ausführungsformen der erfindungsgemäßen Vorrichtung gemäß Fig. 1 bis 5, 15 und 16 ersichtlich. Sie sind teilweise im Vorstehenden bereits erwähnt. Der Kraftmesser 237 gemäß Fig. 1 ist beispielsweise als Biegebalkenmessbrücke ausgestaltet und mißt die Kraftwirkung sowie deren zeitliche Änderung auf den Daumensteg 14 und damit die Kraft am Muskel. Der Kraftmesser 238 an der Hauptgeräteachse 1, beispielsweise als Torsionsmesser, mißt die Kraft an der Hauptgeräteachse 1 zwischen der Kraftquelle 188, 189 bzw. 190 und dem Achsanschlag 125 und damit zugleich die von den Anschlägen unterstützten Nachlasten vor deren Einwirkung auf den Muskel. Der Potentiometer 239 bzw. 239' mißt die Stellung der Hauptgeräteachse 1 und damit die Länge der untersuchten Muskeln und die Geschwindigkeit ihrer Längenänderung. Der Beschleunigungsmesser 240 an der Daumenstegverschiebung 47 bzw. 93 mißt die Beschleunigungen der Muskelkontraktion und der Muskelerschlaffung.

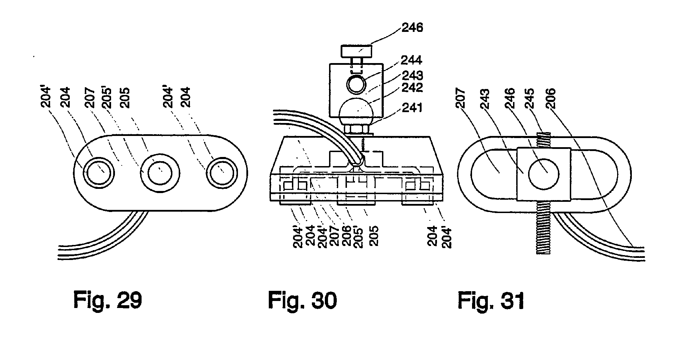

Die anderen Sensoren werden in Kontakt mit der Testperson gebracht. In Fig. 29 bis 31 ist eine Kombination von Sensoren zur Aufnahme elektrischer Potentialschwankungen, der Hauttemperatur und von akustischen Signalen bzw. Vibrationen als einheitliches Bauteil dargestellt. In dem Sensorgehäuse 207 sind auf der unteren, zur Auflage auf den Körper bestimmten Ebene zwei Elektrodenplättchen/ Thermosensoren 204 angeordnet, die der Aufnahme elektrischer Potentialschwankungen und der Hauttemperatur dienen. Sie sind in Elektrodennäpfchen 204' eingesetzt, die zugleich zur Aufnahme von Kontaktmitteln wie z. B. Gel oder Paste dienen. Ferner ist in derselben Ebene ein Vibrationssensor bzw. Mikrophon 205 mit Schallrohr 205' angeordnet, das der Aufnahme von Geräuschen und Vibrationen des untersuchten Körperbereichs dient. Die Sensoren 204, 205 sind durch das kabel 206 mit der, hier nicht dargestellten, Verstärker- und Auswerteelektronik verbunden.

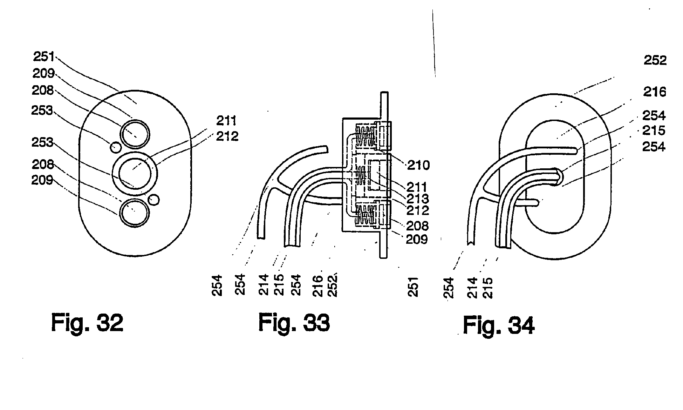

In Fig. 32 bis 34 ist eine abgewandelte Ausführungsform der Sensoreneinheit gemäß Fig. 29 bis 31 dargestellt. Die Sensoren 208, 209 entsprechen grundsätzlich den Sensoren 204, 204' und dienen zur Aufnahme elektrischer Potentialschwankungen und der Hauttemperatur. Der Sensor 211, 212 ist ein Vibrationssensor bzw. Mikrophon wie der Sensor 205, 205'. Diese Sensoren sind jedoch jeweils mit Ausgleichsfedern 210 bzw. 213 im Sensorgehäuse 216 versehen, durch die kleine Unebenheiten oder Veränderungen der Auflagefläche am Untersuchungsobjekt (Haut) unter Erhaltung eines allseitigen definierten Andrucks an diese ausgeglichen werden. Zusätzlich hat die Sensoreinheit auf ihrer Unterseite und wahlweise auch auf ihrer Oberseite selbstklebende Haftflächen 251, 252 zur Anheftung an den untersuchten Körperbereich, die vorzugsweise den gesamten Randbereich der Unterseite der Sensoreinheit bedecken. Außerdem sind durch das Gehäuse von der Unterseite der Sensoreinheit her Bohrungen 253 zum Anschluß der Sensoreinheit an ein Vakuumgerät über Zuleitungsschläuche 254 vorgesehen, die einen sicheren Ansatz der Sensoreinheit am Körper mittels Vakuums ermöglichen.

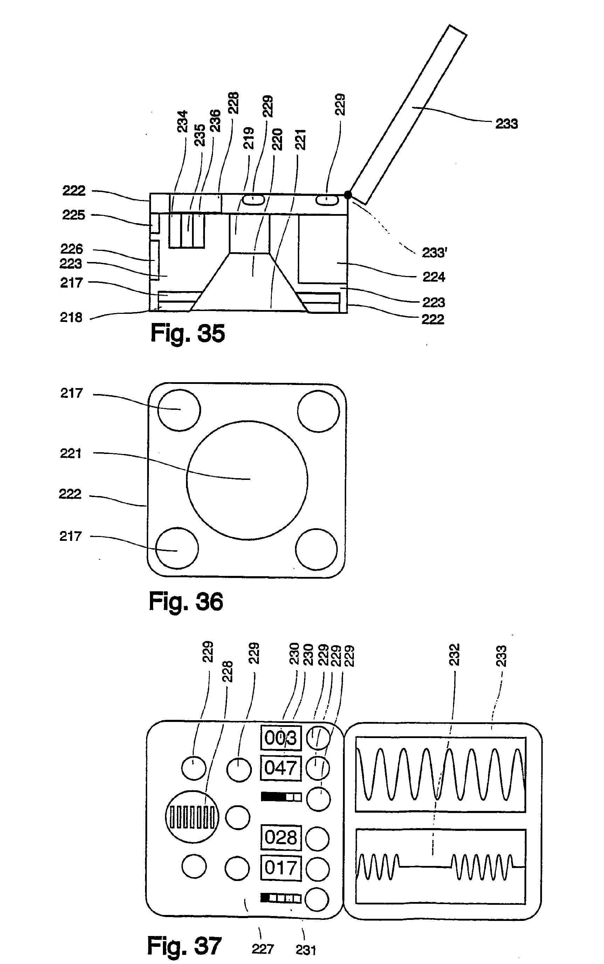

Schließlich ist in den Fig. 35 bis 37 eine Multisensoreinheit 217 bis 236 mit integrierten Vorrichtungen zur Darstellung, Speicherung und Verarbeitung der aufgenommenen Meßwerte gezeigt, die Gegenstand einer gesonderten Patentanmeldung des Anmelders ist und auf deren Beschreibung im einzelnen daher hier verzichtet wird.

Soweit in der vorstehenden Beschreibung Einzelmerkmale der Vorrichtung und ihrer Bauteile, die in den Zeichnungen dargestellt sind als nicht erfindungswesentlich nicht besonders erwähnt werden, wird dazu auf die als Bestandteil der Anmeldung geltende Auflistung der Bezugsziffern der Einzelteile der Vorrichtung verwiesen.

Die beschriebene Vorrichtung nebst Weiterbildungen bringt gegenüber den bekannten Vorrichtungen namentlich folgende Vorteile. Es werden definierbare und vollautomatisiert konstante Experimentaleingriffe am motorischen System und insbesondere an den Muskeln ermöglicht durch exakte und wiederholbare Verschienung bzw. Fixierung, durch definierbare und wiederholbare Muskellängenbegrenzungen und durch konstante und wiederholbare Lasten.

Die Auflage- und Fixierungseinrichtung mit Verschienungsauflage und mit Armauflagentisch in der beschriebenen Ausgestaltung ermöglicht die Wiederholbarkeit der Untersuchung durch Wiederpositionierung der Auflage- und Fixierungseinrichtung und des zu untersuchenden Körperteils durch automatische Einstellung der früher gemessenen und gespeicherten Werte.

Die Vorrichtung ermöglicht die Untersuchung eines Körperteils, insbesondere der menschlichen Hand, in verschiedenen Positionen, indem eine Daumenschale vor oder hinter dem Daumen stützend angebracht sein kann, je nachdem, ob ein größerer Bewegungsspielraum zwischen Daumen und Hand nach hinten oder nach vorn sein soll. Ebenso kann die Extremität mit der Handfläche nach oben oder nach unten fixiert bzw. eingeschient werden, je nachdem wie die Beweglichkeit der Untersuchungsperson ist und ob die Handfläche oder der Handrücken besser zugänglich sein sollen. Ferner ist bei derselben Fixierung eine Untersuchung des Agonist wie z. B. des Daumenadduktors und des Antagonist wie z. B. des Daumenabduktors möglich. Darüber hinaus wird durch die Vorrichtung die Untersuchung der rechten und/oder der linken Extremität wahlweise möglich und können unterschiedliche Bewegungsachsen, somit verschiedene Muskelgruppen untersucht werden.

Durch die erfindungsgemäßen automatisierten Anschläge der Vorrichtung werden Muskellängenveränderungen unter eine bestimmte Länge und/oder über eine bestimmte Länge verhindert, womit die Muskellängenbedingungen vor Beginn des Experiments auf den Punkt genau festgelegt werden können.

Durch die Positionierung der beschriebenen Anschläge kann außerdem der Kontraktionstyp bestimmt werden, nämlich ob der Muskel eine reine Kraftkontraktion (isometrisch) durchführt oder ob eine reine Verkürzung eintritt (isotonisch) oder ob der Muskel eine Unterstützungszuckung oder eine Anschlagszuckung ausführt.

Durch die beschriebenen Kraftquellen und ihrer Ausgestaltung können definierbare Lasten auf den Muskel wirken und diese über die Anschläge zu Vor- und Nachlasten werden. Das Drehmoment der Kraftquelle wird so geregelt, daß Fehlerquellen durch die Masse von bewegten Teilen, Drehmomentschwankungen, Elastizitäten und Reibungausgeschlossen oder ausgeglichen werden. Die beschriebenen Kraftquellen haben ein großes Leistungsspektrum und sind weitgehend wartungsfrei.

Durch den beschriebenen Aufbau der sich exzentrisch an der Hauptgeräteachse bewegenden Teile werden Verzerrungen des Versuchsablaufs vermieden und über den gesamten Versuchszeitablauf konstante Bedingungen erreicht. Insbesondere treten keine positionsabhängigen Schwerkraftwirkungen und kaum beschleunigungsabhängige Drehmomentsveränderungen, bewegungsabhängige Drehmomentsvertuste und/oder Schwingungen auf.

Durch die Kraftquelle können selbst Kraftpulse abgegeben werden, die über Reflexbögen selbst zu unwillkürlichen standardisierten Muskelkontraktionen oder- entspannungen führen. Nicht zuletzt können durch die verwandten Reizgeber in Form von Reizelektroden als Magnetspulenstimulator oder Strompulsstimulator unwillkürliche, standardisierte Muskelkontraktionen ausgelöst werden.

### BEZUGSZEICHENLISTE

- 1.: Hauptgeräteachse
- 2.: Bewegungsachse Testperson (2.' Alternative Bewegungsachse)

- 3.: Armauflage Seite (3.' Arm/Handauflage Seite)
- 4.: Armauflage Unterseite
- 5.: Verschraubung Armauflage
- 6.: Handrückenstütze
- 7.: Handrückenpolster
- 8.: Verschiebungssteg (8.' Drehgelenk Handrückenstütze)
- 9.: Handflächenstütze
- 10.: Handgelenksstütze
- 11.: Fingerstütze
- 12.: Verschienungsauflage
- 13.: Daumenschale
- 14.: Daumensteg
- 15.: Daumenhebel

- 16.: Verschieblicher Armauflagentisch (16.' Daumenausschnitt)
- 17.: Armanlage Seite
- 18.: Armstütze Seite
- 19.: Fingerstütze
- 20.: Fingerspitzenstütze
- 21.: Armklemmpolster Unterseite
- 22.: Handflächenklemmpolster
- 23.: Daumenschale
- 24.: Daumensteg
- 25.: Daumenhebel
- 26.: Fixierungsband Daumen

- 27.: Gußschale
- 28.: Abstandsstifte (28.' Abstandslöcher)
- 29.: Rohgußform (29.' Bearbeitete Gußform)
- 30.: Daumenausschnitt
- 31.: Fixierungsband Hand

- 32.: Auflageplatte
- 33.: Achsenhaltering
- 34.: Halteringarretierung
- 35.: Stützklotz (35.' Stützklotzarretierung)
- 36.: Fixierungsband

- 37.: Lichtpeilung Hauptgeräteachse (37.' Blende)
- 38.: Verschiebungsmotor Verschiebungstisch
- 39.: Spindel Verschienungstisch (39.' Spindelführungen)
- 40.: Führungsschienen Verschienungstisch (40.' Schienenhalterungen)
- 41.: Motor Handrückenstütze
- 42.: Spindel Handrückenstütze (42.' Spindelführungen)
- 43.: Verschiebeklötzchen (43.' Langloch)
- 44.: Verschiebungsschiene Handgelenksstütze (44.' Arretierung)
- 45.: Verschiebungsführung Fingerstütze
- 46.: Verschiebungsklötzchen Fingerstütze (46.' Arretierung)
- 47.: Verschiebung Daumensteg (47.' Arretierung)

- 48.: Laserpeilung Schwenkarm
- 49.: Schwenkarm
- 50.: Drehsteg
- 51.: Befestigung Schwenkarm
- 52.: Verschiebungsklötzchen Armauflagentisch
- 53.: Spindel Armauflage Oberseite
- 54.: Spindelführungen
- 55.: Übersetzungsriemen
- 56.: Motor
- 57.: Untergriffschiene Armauflagentisch
- 58.: Führung Armauflage (des Verschienungsgehäuses)
- 59.: Halterung Armstütze Seite
- 60.: Halterungsschrauben
- 61.: Drehgelenk Armstütze Seite (61.' Drehgelenksstift)
- 62.: Langloch Armstütze Seite
- 63.: Verschiebungsklötzchen Armstütze Seite
- 64.: Spindel mit gegenläufigen Gewinden
- 65.: Spindelführungen
- 66.: Übersetzungsriemen
- 67.: Motor
- 68.: Drehgelenk Fingerstütze (68.' Drehgelenksstift)
- 69.: Langloch Fingerstütze
- 70.: Verschiebungsklötzchen Fingerstütze
- 71.: Spindel mit gegenläufigen Gewinden
- 72.: Spindelführungen
- 73.: Übersetzungsriemen
- 74.: Motor
- 75.: Verschiebungsklötzchen Fingerspitzenstütze
- 76.: Spindel Fingerspitzenstütze
- 77.: Spindelführungen
- 78.: Übersetzungsriemen
- 79.: Langloch Fingerspitzenstütze
- 80.: Motor
- 81.: Teleskopstange
- 82.: Bügelbefestigung
- 83.: Befestigungslöcher
- 84.: Befestigungsschrauben
- 85.: Haltebügel Armklemmpolster
- 86.: Verschiebungsstab Armklemmpolster
- 87.: Arretierung Armklemmpolster
- 88.: Bügelbefestigung
- 89.: Befestigungslöcher (89.'Befestigungsschrauben)
- 90.: Haltebügel Handflächenklemmpolster
- 91.: Verschiebungsstab Handflächenklemmpolster
- 92.: Arretierung Handflächenklemmpolster
- 93.: Verschiebung Daumensteg
- 94.: Arretierung Verschiebung Daumensteg
- 95.: Laserpeilung Hauptgeräteachse

- 96.: Schwenkarm mit Laserpeilung
- 97.: Langloch Stützklotzverschiebung

- 98.: Strommesser für Verschienungsmotoren

- 99.: Meßskala Verschiebung Daumensteg
- 100.: Linearmesser Position Armtisch
- 101.: Verbindungsstück Armtischmesser
- 102.: Linearmesser Position Armstütze
- 103.: Verbindungsstück Armstützemnesser
- 104.: Linearmesser Position Fingerstütze
- 105.: Verbindungsstück Fingerstützenmesser
- 106.: Linearmesser Position Fingerspitzenstütze
- 107.: Verbindungsstück Fingerspitzenstützenmesser

- 108.: Meßskala Stützklotzverschiebung

- 109.: Reizelektroden (-näpfchen)
- 110.: Platte Reizelektrodenpaar
- 111.: Griffmulden
- 112.: Kabel Reizelektrodenpaar (112. Gesamtkabel)
- 113.: Stecker Reizelektrodenpaar (113.' Gesamtstecker)
- 114.: Schaltkästchen
- 115.: Einzelschalter Reizelektrodenpaar (115.' Gesamtschalter)

- 116.: Halterklötzchen Reizelektrodenplatte (116.' Halterklötzchen anderer Höhe)
- 117.: Halterlippe (117.' Halterlippe für gleichseitiges Fixieren) (117." Halterlippe mit Schlitzen)
- 118.: Fixierungsbandlaschen
- 119.: Fixierungsband (119.` Kantenverstärkung Fixierungsband)
- 120.: Verschluß
- 121.: Verschlußöffner
- 122.: Klemmungsachse
- 123.: Klemmung
- 124.: Klemmungsfeder

- 125.: Achsenanschlag Rotation
- 126.: Vorderer Rotationsanschlag

- 127.: Vorderer Achsenanschlag Linearbewegung
- 128.: Vorderer Linearanschlag

- 129.: Hinterer Rotationsanschlag

- 130.: Hinterer Achsenanschlag Linearbewegung
- 131.: Hinterer Linearanschlag (=237.' Kraftmesser Linearachse)

- 132.: Linearachse
- 133.: Übersetzungsrad
- 134.: Übersetzungsseilachse (134. Übersetzungsseil Potentiometer)
- 135.: Gegenzugseil
- 136.: Seilklemmung Linearachse (136.' Gegenzugseilklemmung)
- 137.: Klemmungssteg
- 138.: Obere Linearachsenführung
- 139.: Führungssteg
- 140.: Gehäuse
- 141.: Gehäusezwischenwände
- 142.: Gehäusedoppelwand

- 143.: Vorderer Rotationsanschlag Verstellrad
- 144.: Verstellradlager
- 145.: Führungsrohr
- 146.: Zahnkranz Verstellrad
- 147.: Zahnrad Verstellmotor
- 148.: Achse Verstellmotor
- 149.: Verstellmotor
- 150.: Halterung Verstellmotor

- 151.: Gleitlager vorderer Linearanschlag
- 152.: Führungsstange
- 153.: Haltesteg Führungsstange
- 154.: Bewegungsspindel vorderer Linearanschlag
- 155.: Linearanschlagsgewinde für Bewegungsspindel
- 156.: Durchlaß Bewegungsspindel
- 157.: Lager Bewegungsspindel
- 158.: Motor Bewegungsspindel

- 159.: Hinterer Rotationsanschlag Verstellrad
- 160.: Verstellradlager
- 161.: Führungsrohr
- 162.: Zahnkranz Verstellrad
- 163.: Zahnrad Verstellmotor
- 164.: Achse Verstellmotor
- 165.: Verstellmotor

- 166.: Gleitlager hinterer Linearanschlag
- 167.: Führungsstange
- 168.: Haltesteg Führungsstange
- 169.: Bewegungsspindel hinterer Linearanschlag
- 170.: Linearanschlagsgewinde für Bewegungsspindel
- 171.: Durchlaß Bewegungsspindel
- 172.: Lager Bewegungsspindel
- 173.: Motor Bewegungsspindel

- 174.: Potentiometerachse vorderer Rotationsanschlag
- 175.: Potentiometer vorderer Rotationsanschlag

- 176.: Übersetzungsrad Spindel für vorderen Linearanschlag
- 177.: Übersetzungsseil
- 178.: Übersetzungsrad Potentiometer
- 179.: Potentiometerachse
- 180.: Potentiometer vorderer Linearanschlag

- 181.: Potentiometerachse hinterer Rotationsanschlag
- 182.: Potentiometer hinterer Rotationsanschlag

- 183.: Übersetzungsrad Spindel für hinteren Linearanschlag
- 184.: Übersetzungsseil
- 185.: Übersetzungsrad Potentiometer
- 186.: Potentiometerachse
- 187.: Potentiometer hinterer Linearanschlag

- 188.: Drehmagnet 1
- 189.: Drehmagnet 2

- 190.: (Hall-)Motor

- 191.: Linearantrieb (Linearmotor oder Hubmagnet)
- 192.: Linearachsenkern

- 193.: Drehmagnetlager
- 194.: Drehmagnetzahnkranz
- 195.: Zahnstange
- 196.: Zahnstangenlager
- 197.: Zahnrad (197.'Übersetzung Zahnrad)
- 198.: Übersetzungsriemen
- 199.: Motor Drehmagnetverstellung
- 200.: Halteplatte

- 201.: Potentiometer Drehmagnetverstellung

- 202.: Einstellbare Rutschkupplung

- 203.: Bolzen

- 204.: Elektrodenplättchen / Thermosensor (204.' Elektrodennäpfchen)
- 205.: Vibrationssensor / Mikrophon (205.' Schallrohr)
- 206.: Kabel
- 207.: Sensorgehäuse

- 208.: Elektrodenplättchen / Thermosensor
- 209.: Elektrodennäpfchen
- 210.: Ausgleichsfeder Elektrodennäpfchen
- 211.: Vibrationssensor (Mikrophon)
- 212.: Vibrationssensorbefestigung
- 213.: Ausgleichsfeder Vibrationssensorbefestigung
- 214.: Elektrodenkabel
- 215.: Vibrationssensorkabel
- 216.: Sensorgehäuse

- 217.: Elektrodenplättchen / Thermosensor
- 218.: Elektrodennäpfchen
- 219.: Vibrationssensor (Mikrophon)
- 220.: Schalltrichter
- 221.: Membran
- 222.: Sensorgehäuse mit Abschirmung
- 223.: Raum für digitale und analoge Elektronik
- 224.: Akkumulator bzw. Schittstelle zum Netzstrom
- 225.: Schnittstelle zum Ohr-/Kopfhörer
- 226.: Schnittstelle zum Computer
- 227.: Bedienfeld
- 228.: Lautsprecher
- 229.: Multifunktionale Bedientasten
- 230.: Displaydigitalfelder
- 231.: Displayamplitudenbalken
- 232.: Displaykurvenfelder
- 233.: Displaydeckel (233.'Deckelscharnier)
- 234.: Triggerelement
- 235.: Datenspeicherelement
- 236.: Filterelement

- 237.: Kraftmesser Daumensteg (237.' Kraftmesser Linearachse)

- 238.: Kraftmesser Hauptgeräteachse

- 239.: Potentiometer Hauptgeräteachse (239.' Potentiometer Linearachse)

- 240.: Beschleunigungsmesser

- 241.: Gehäusehalterung
- 242.: Gelenkkugel Gehäusehalterung
- 243.: Klötzchen für Verschiebungsfeder mit Gelenkpfanne
- 244.: Führungskanal Verschiebefeder zur Querverschiebung
- 245.: Elastische Verschiebefeder zur Querverschiebung
- 246.: Klemmschraube Verschiebefeder
- 247.: Längsloch Verschienungstisch
- 248.: Linearachse zur Längsverschiebung
- 249.: Klemmführung für Linearachse
- 250.: Klemmhebel für Linearachse

- 251.: Innerer Kleberand
- 252.: Äußerer Kleberand
- 253.: Vakuumkanäle
- 254.: Vakuumschläuche

- 255.: Integriertes Masseband

## Patentansprüche

1. Vorrichtung zur Untersuchung des motorischen Systems des menschlichen oder tierischen Körpers durch Untersuchung und Bestimmung der Funktions- und Leistungsfähigkeit der Muskulatur, mit einer Auflage- und Fixierungseinrichtung mit Verschienungsauflage oder mit Armauflagentisch und mit einer Körpergliedhalterung, insbesondere in Form einer Daumenschale mit Daumensteg und Daumenhebel, zur Einsetzung, Auflage und Fixierung der zu untersuchenden Körperteile, wie zum Beispiel der Hand und des Unterarms, mit Mitteln zur wiederholten Untersuchung derselben Körperteile in derselben fixierten Position mit einer Kraftquelle zur Belastung der Muskeln sowie mit Reizgebern zur Stimulation/Kontraktion von Muskel, Nerv oder zentralem Nervensystem und mit mechanischen und elektrisch-elektronischen Meßgeräten zur Messung von Kraft, Beschleunigung und Muskellängen, **dadurch gekennzeichnet, daß** der Daumenhebel (15, 25) mit der Daumenschale (13, 23) und dem Daumensteg (14, 24) mit der senkrecht zum Daumenhebel (15, 25) angeordneten Hauptgeräteachse (1) kraftschlüssig verbunden ist und diese Hauptgeräteachse (1) mit der Kraftquelle in Form von einem oder zwei Drehmagneten (188, 189) oder eines Elektromotors (190) oder eines Linearantriebs (Linearmotor, Hubmagnet) (191) in Verbindung steht bzw. in die Kraftquelle (188, 189; 190; 191) eingreift, daß ferner zur Festlegung der Art der Kontraktion und zur automatisierten Kraft- und/oder Längenbegrenzung die Hauptgeräteachse (1) zwischen der Verbindung mit dem Daumenhebel (15; 25) und der Kraftquelle (188, 189; 190) den mit einem vorderen Rotationsanschlag (126) und einem hinteren Rotationsanschlag (129) zusammenwirkenden Achsanschlag (125) hat, daß die StimulatioNKontraktion von Muskel, Nerv oder zentralem Nervensystem durch die Kraftquelle (188, 189; 190;191) oder durch periphere elektrische Stimulation mittels Reizgebern (109, 110) erfolgt, daß die Kraftmesser (237, 238) die Kraftwirkung auf den Daumensteg (14; 24) sowie deren zeitliche Änderung und die Kraft an der Hauptgeräteachse (1) zwischen der Kraftquelle (188, 189; 190) und dem Achsanschlag (125) messen, während die Messung der Beschleunigung der Muskelkontraktion durch einen Beschleunigungsmesser (240) an der Daumenstegverschiebung (47; 93) des Daumenstegs (14; 24) erfolgt und der Potentiometer (239; 239') durch die Messung der Stellung der Hauptgeräteachse (1) die Muskellänge mißt, und daß Sensoren zur Aufnahme von elektrischen Potentialschwankungen, der Hauttemperatur und von akustischen Signalen und Vibrationen vorhanden sind.

2. Vorrichtung nach Patentanspruch 1, **dadurch gekennzeichnet, daß** der mit dem Achsanschlag (125) zusammenwirkende vordere Rotationsanschlag (126) auf einem Verstellrad (143) angebracht und über dieses im Winkel verstellbar ist und der hintere Rotationsanschlag (129) auf einem Verstellrad (159) angebracht und über dieses ebenfalls im Winkel verstellbar ist.

3. Vorrichtung nach Patentanspruch 2, **dadurch gekennzeichnet, daß** das Verstellrad (143) mittels eines Lagers (144) auf einem Führungsrohr (145) gelagert ist, das die Hauptgeräteachse (1) ohne Kraftschluß umgreift, und ferner auf der anschlagsfernen Seite mit einem Zahnkranz (146) versehen ist, in den ein Zahnrad (147) eingreift, das seinerseits über die Achse (148) von einem Verstellmotor (149) angetrieben wird, ferner **dadurch gekennzeichnet, daß** das Verstellrad (159) mittels eines Lagers (160) auf dem Führungsrohr (161) gelagert ist, das die Haüptgeräteachse (1) ohne Kraftschluß umgreift, und das Verstellrad (159) auf der anschlagsfernen Seite mit den Zahnkranz (162) versehen ist, in den ein Zahnrad (163) eingreift, das über eine Achse (164) von einem Verstellmotor (165) angetrieben wird, wobei am Verstellmotor (149) über eine Achse (174) ein Potentiometer (175) zur Messung der Stellung des vorderen Rotationsanschlags (126) und am Verstellmotor (165) über eine Achse (181) ein Potentiometer (182) zur Messung der Stellung des hinteren Rotationsanschlags (129) angesetzt sind.

4. Vorrichtung nach Patentanspruch 1, **dadurch gekennzeichnet, daß** bei Verwendung der Kraftquelle (191) in Form eines Linearmotors oder Hubmagneten zwischen dieser und ihrer Verbindung mit der Hauptgeräteachse (1) ein vorderer Achsanschlag (127) auf der Linearachse (132) im Anschluß an die Kraftquelle fest angebracht ist, der den Anschlag für den quer zur Linearachse (132) angeordneten vorderen Linearanschlag (128) bildet, und daß ferner ein hinterer Achsanschlag (130) auf der Linearachse (132) fest angebracht ist, der den Anschlag für den ebenfalls quer zur Linearachse (132) angeordneten hinteren Linearanschlag (131) bildet, wobei die Linearanschläge (128, 131) jeweils aus einem Steg bestehen, der jeweils durch senkrecht zu ihnen stehende Führungsstangen (151, 167) geführt wird und an diesen jeweils über Gleitlager (151, 166) parallel zur Linearachse (132) verschieblich ist, und wobei die Verschiebung des Linearanschlags (128) durch einen Motor (158) über eine Spindel (154) und die Verschiebung des Linearanschlags (131) durch einen Motor (173) über eine Spindel (169) erfolgen, und daß an der Spindel (154) ein Potentiometer (180) zur Messung der Stellung des vorderen Linearanschlags (128) und an der Spindel (169) ein Potentiometer (187) zur Messung der Stellung des hinteren Linearanschlags (131) angeordnet sind.

5. Vorrichtung nach Patentanspruch 1, **dadurch gekennzeichnet, daß** bei der Auflage- und Fixierungseinrichtung mit der Verschienungsauflage (12) diese senkrecht auf einer Gehäusequerwand (142) des Gehäuses (140, 141, 142) der Vorrichtung angesetzt ist und an ihr eine Armauflage (3) mit anschließender Handauflage (3') angebracht sind, daß ferner im rechten Winkel zur Verschienungsauflage (12) und zur Armauflage (3) die Handrückenstütze (6) mit Handrückenpolster (7) und Verschiebungssteg (8) angeordnet und über diesen Verschiebungssteg (8) in einer angepassten Ausnehmung (43') der Verschienungsauflage (12) vertikal verschiebbar sind, daß ferner ebenfalls im rechten Winkel zur Armauflage (3) eine Armauflage (4) vorhanden ist und auf der Handauflage (3') eine Handgelenkstütze (10) mittels einer Träger- und Verschiebungsschiene (44) horizontal verschiebbar und durch eine Befestigungsschraube (44') arretierbar angesetzt ist, daß außerdem eine Handflächenstütze (9) im Anschluß an die Handgelenkstütze (10) und in einer Ebene mit dieser verläuft und eine Fingerstütze (11) für die Finger II - V verschieblich angebracht ist, wobei die Fixierung der eingeführten Hand nebst Handgelenk derart erfolgt, daß die Handrückenstütze (6) nebst Handrückenpolster (7) durch einen Elektromotor (41) mit Strommesser/-abschalter (98) über eine Spindel (42), in die der Verschiebungssteg (8) mittels eins Verschiebeklötzchens (43) eingreift, vertikal verschoben werden, und daß anderseits die Handgelenkstütze (10) auf der Verschiebungsschiene (44) horizontal verschoben und mittels der Stellschraube (44') arretiert wird, wobei der Motor (41) und die Spindel (42) auf der der Armauflage (3) abgewandten Seite der Verschienungsauflage (12) mittels Spindelführungen (42') angeordnet sind, und daß die Handrückenstütze (6) mit dem Verbindungssteg (8) über ein Drehgelenk (8') im Bereich des Eingriffs in die Spindel um eine vertikale Achse schwenkbar ist sowie die Fingerstütze (11) mittels eines Verschiebeklötzchens (46) auf einer Verschiebungsführung (45) an den Zeigefinger herangeführt und mit den Stellschrauben (46') arretiert wird.

6. Vorrichtung nach einem oder mehreren der Patentansprüche 1 bis 5, **dadurch gekennzeichnet, daß** die Verschienungsauflage (12) horizontal und parallel zu der Zwischenwand (142) des Gehäuses der Vorrichtung auf einer oder mehreren Führungsschienen (40), die mit Halterungen (40') an der Zwischenwand angebracht und als Spindeln ausgestaltet sind, mittels eines Elektromotors (38) verschieblich ist.

7. Vorrichtung nach Patentanspruch 1, **dadurch gekennzeichnet, daß** bei der Auflage- und Fixierungseinrichtung mit Armauflagentisch (16, 16') dieser in Führungen (58) horizontal verschieblich parallel zur Gehäusequerwand (142) über dieser angeordnet ist, in den der Armauflagentisch (16) durch einen Motor (56) der unterhalb des Armauflagentischs auf der Gehäusequerwand (142) montiert ist über ein Verbindungsklötzchen (52), das fest an der Unterseite des Armauflagentischs (16) angesetzt ist und in einer Spindel (53) mit Spindelführungen (54) an der Unterseite des Armauflagentischs geführt ist und über eine Übertragungsriemen (55) zur Spindel (53) verschoben wird, ferner **dadurch gekennzeichnet, daß** mittig eine Armanlage (17) zur beidseitigen Anlage des aufgelegten Arms nebst Hand angebracht ist, an deren Oberseite ein Armklemmpolster (21) mit Haltebügel (85) und Bügelbefestigung (82) sowie im Abstand von diesem ein Handflächenklemmpolster (22) mit Haltebügel (90) und Bügelbefestigung (88) angesetzt sind, die jeweils in Längsrichtung der Armanlage (17) mit Schrauben (84, 89') in Lochreihen (83, 89) versetzbar sind, wodurch das Armklemmpolster (21) und das Handflächenklemmpolster (22) auch jeweils um 180° versetzbar sind zur Fixierung des jeweils eingelegten Arms nebst Hand, wobei das Armklemmpolster mittels eines Verschiebungsstabs (86) mit Arretierung (87) und das Handflächenklemmpolster (22) mittels eines Verschiebungsstabs (91) mit Arretierung (92) höhenverschieblich zur Anpassung an Handfläche und Arm sind, ferner daß beidseits der Armanlage (17) jeweils auf dem Armauflagentisch (16) eine Fingerstütze (19) verschieblich angeordnet ist, die ein Drehgelenk (68) mit Drehgelenkstift (68') hat und über diesen und ein Langloch (69) im Armauflagentisch (16) mit einem Verschiebeklötzchen (70) verbunden ist, das durch eine Spindel (71) mit gegenläufigen Gewinden und einen Motor (74) mit Übersetzungsriemen (73) für die Spindel die Fingerstützen (19) gegenläufig zur Verschienung an die jeweils eingesetzte Hand führt, und wobei die Spindel (71) mit Spindelführungen (72) ebenso wie der Motor (74) an der Unterseite des Armauflagentischs (16) befestigt sind, wobei ferner entsprechend den Fingerstützen (19) zur Abstützung der Außenseite des Arms im Bereich des Armklemmpolsters (21) beidseits der Armanlage (17) Armstützen (18) mit dem Drehgelenk (61), dem Drehgelenkstift (61'), dem Verschiebeklötzchen (63), der Spindel (64) mit Spindelführungen (65) und dem Motor (67) mit Übersetzungsriemen (66) vorgesehen sind, die an den aufgelegten Arm herangeführt werden.

8. Vorrichtung nach Patentanspruch 7, **dadurch gekennzeichnet, daß** zur Abstützung der Fingerspitzen bei der Auflage- und Fixierungseinrichtung mit Armauflagentisch (16) auf diesem eine verschiebliche Stütze (20) angeordnet ist mit einem auf dem Armauflagentisch in einer Spindel (76) mit Spindelführungen (77) geführten Verschiebeklötzchen (75), die durch einen Motor (80) mit Übersetzungsriemen (78) bewegt werden und die Stütze (20) mit Teleskopführungen (81) an die Fingerspitzen der untersuchten Hand zur Fixierung heranführen.

9. Vorrichtung nach einem oder mehreren der Patentansprüche 1 bis 7, **dadurch gekennzeichnet, daß** die Auflage- und Fixierungseinrichtungen (3 bis 12) bzw. (16 bis 22) als abtrennbare Module der Vorrichtung gegeneinander oder gegen andere Verschienungseinheiten ausgetauscht werden können, wobei sie vorzugsweise durch eine Gehäusedoppelwänd (142) von der übrigen Vorrichtung getrennt sind.

10. Vorrichtung nach einem oder mehreren der Patentansprüche 1 bis 8, **dadurch gekennzeichnet, daß** für den zu untersuchenden Finger oder Daumen der eingeschobenen Hand eine Daumenschale (13) mit Daumensteg (14) bzw. eine Daumenschale (23) mit Daumensteg (24) vorhanden sind, wobei der Daumensteg (14; 24) in den horizontal angeordneten Daumenhebel (15; 25) eingreift und auf diesem mittels der Verschiebevorrichtung (47; 93) mit Arretierungen (47', 94) horizontal verschiebbar ist.

11. Vorrichtung nach einem oder mehreren der Patentansprüche 1 bis 10, **dadurch gekennzeichnet, daß** am obersten Ende der Hauptgeräteachse (1) ein Lichtstrahler, vorzugsweise ein Laserpeilstrahler (37) mit Blende (37') eingesetzt ist, dessen Peilstrahl auf das zu untersuchende Körperteil, wie zum Beispiel die Hand, gerichtet und mit dessen Bewegungsachse vor seiner Fixierung zur Deckung gebracht wird.

12. Vorrichtung nach einem oder mehreren der Patentansprüche 1 bis 10, **dadurch gekennzeichnet, daß** anstelle oder zusätzlich zum Laserpeilstrahler (37) gemäß Fig. 10 ein Laserpeilstrahler (48) am einen Ende eines Schwenkarms (49) angeordnet ist, der mit seinem anderen Ende an der Führung (58) des Armauflagentischs (16) drehbar eingesetzt ist und über das aufgelegte zu untersuchende Körperteil geschwenkt wird, wobei der Laserpeilstrahler (48) und sein Peilstrahl in der eingeschwenken Position in der gedachten Verlängerung der Hauptgeräteachse (1) liegen und mit dieser übereinstimmen, und wobei die Bewegungsachse des zu untersuchenden Körperteils vor dessen Fixierung mit dem Peilstrahl zur Deckung gebracht wird.

13. Vorrichtung nach den Patentanspruch 1 und einem oder mehreren der Patentansprüche 2, 3, 4, 10, 11 und 12, **dadurch gekennzeichnet, daß** bei der Auflage- und Fixierungseinrichtung mit Armauflagentisch (16, 16'), der auf den Führungen (58) horizontal verschieblich und parallel zur Gehäusequerwand (142) angeordnet ist, eine Gußform (29) als Negativform zur Auflage- und Fixierung der zu untersuchenden Hand aus Kunststoff oder anderem Material mittels eingegossenen, aus der Unterseite der Gußform (29) hervorstehenden Abstandsstiften (28) in angepasste Bohrungen (28') des Armauflagentischs (16) so eingesetzt wird, daß die Bewegungsachse der Hand mit der Hauptgeräteachse der Vorrichtung zur Deckung kommt, und die Gußform (29) im Bereich des Daumens und des Daumenmuskels einen Ausschnitt (30) für die Daumenschale (23) mit Daumensteg (24) zur Anlegung des Daumens und Verbindung zum Daumenhebel (25)und zur Hauptgeräteachse (1) hat.

14. Vorrichtung nach Patentanspruch 13, **dadurch gekennzeichnet, daß** zur Herstellung der Gußform (29) eine Gußschale (27) mit in deren Boden lösbar eingesteckten Abstandsstiften (28) dient und an einem der oberen Außenränder der Gußschale (27) ein Laserpeilstrahler mit Schwenkarm (96) angeordnet ist, wobei zur Herstellung der Gußform (29) die Hand mit dem Handrücken auf die Abstandsstifte (28) gelegt, der Laserpeilstrahler (96) darüber geschwenkt und sein Peilstrahler mit der Bewegungsachse der Hand zur Deckung gebracht sowie die Gußschale (27) mit der Hand mit geeignetem Kunststoff oder sonstigem aushärtendem Gießmaterial ausgegossen wird.

15. Vorrichtung nach Patentansprüchen 1 bis 4, **dadurch gekennzeichnet, daß** sie eine Auflageplatte (32) zur Auflage und Fixierung von Körperteilen mit großen Gelenken, wie z. B. das Sprunggelenk hat, auf deren Unterseite ein Haltering (33) mit Halteringarretierung (34) zur kraftschlüssigen Befestigung an der Hauptgeräteachse (1) angebracht ist und daß die Auflageplatte (32) über einen Teil ihrer Länge ein Langloch (97) besitzt, in dem ein auf der Oberseite der Auflageplatte angeordneter Stützklotz (35) verschieblich und mit der Verschraubung (35') arretierbar ist zum Ansatz und zur Fixierung des Körperteils mit seiner Bewegungsachse über der Hauptgeräteachse (1), wobei zusätzlich ein Fixierungsband (36) an der Auflageplatte (32) befestigt ist und eine Meßskala (108) am Langloch (97) zur Messung der Position des untersuchten Körperteils angeordnet ist.

16. Vorrichtung nach Patentanspruch 1 und einem oder mehreren der Patentansprüche 2, 3, 5 bis 15, **dadurch gekennzeichnet, daß** als Kraftquelle ein Drehmagnet (188) mit konstanter oder konstant steigender Kennlinie dient.

17. Vorrichtung nach Patentanspruch 1 und einem oder mehreren der Patentansprüche 2, 3, 5 bis 15, **dadurch gekennzeichnet, daß** als Kraftquelle zwei Drehmagnete (188, 189), vorzugsweise mit in Summe konstanter oder konstant steigender Kennliene, dienen, die hintereinander geschaltet sind und um den mit der Hauptgeräteachse (1) verbundenen Innenteil rotiert werden, indem zwischen den Drehmagneten (188, 189) ein Zahnkranz (194) angeordnet ist, der über eine Zahnstange (195) mit Zahnstangenlager (196), ein Zahnrad (197) und einen Übersetzungsriemen (198) mit einem Motor (199) in Verbindung steht und mittels dieses die Drehmagnete (188, 189) verstellt, und daß am Motor (199) ein Potentiometer (201) zur Messung der Stellung der Drehmagnete (188, 189) angesetzt ist.

18. Vorrichtung nach Patentanspruch 1 und einem oder mehreren der Patentansprüche 2, 3, 5 bis 15, **dadurch gekennzeichnet, daß** als Kraftquelle ein Elektromotor (190), vorzugsweise in Form eines Hallmotors, dient.

19. Vorrichtung nach Anspruch 1 und einem oder mehreren der Patentansprüche 4 bis 15, **dadurch gekennzeichnet, daß** als Kraftquelle ein Linearantrieb (191) in Form eines Linearmotors oder Hubmagneten dient, der über die Linearachse (132) und eine Übersetzung/Kraftumlenkung (133, 134, 135, 136, 137) Kraft auf die rechtwinklig zur Linearachse angeordnete Hauptgeräteachse (1) überträgt, wobei ein Übersetzungsrad (133) fest auf der Hauptgeräteachse (1) im Bereich der Linearache (132) sitzt mit einem Übersetzungsseil (134), dessen eine Hälfte oben über das Übersetzungsrad (133) zu einer unteren Seilklemmung (136) auf der Linearachse (132) läuft und fixiert wird und dessen andere Hälfte unten über das Übersetzungsrad (133) zu einer oberen Seilklemmung (136) auf der Linearachse (132) läuft und fixiert wird, und daß auf der dem Übersetzungsseil (134) gegenüberliegenden Seite der Linearachse (132) ein Gegenzugseil (135) mit Klemmungen (136'), die ihrerseits über Klemmungsstege (137) an der Linearachse (132) befestigt sind, zur Verhinderung der einseitigen Belastung und Durchbiegung der Linearachse (132) angebracht ist, und wobei die Linearachse mit ihrem der Kraftquelle (191) gegenüberliegenden Ende in einem Führungssteg (139) mit Gleitlager (138) gelagert ist.

20. Vorrichtung nach Patentanspruch 1 oder einem oder mehreren der Ansprüche 2 bis 19, **dadurch gekennzeichnet, daß** die Stimulation des motorischen Systems in Form von willkürlicher Auslösung, zentraler oder peripherer Magnetstimulation, Reflexstimulation vorzugsweise durch Änderung der Muskellänge durch die Kraftquelle oder als periphere elektrische Stimulation durch Reizgeber mit Reizelektroden erfolgt.

21. Vorrichtung nach Patentanspruch 20, **dadurch gekennzeichnet, daß** die Reizelektroden als Zwillings-Reizelektrodenpaar (109, 110) ausgebildet sind, bei dem jeweils in einer Träger- und Halteplatte (110) zwei Reizelektroden (109) eingesetzt sind und jedes Elektrodenpaar über Kabel und Stecker (112, 113) an ein Schaltkästchen (114) angeschlossen ist, das für jedes Reizelektrodenpaar Einzelschalter 115 und einen Gesamtschalter (115') aufweist, und daß die Reizelektrodenpaare mit der Steuerungselektronik der Steuerungsvorrichtung über Kabel und Stecker (112', 113') verbunden sind.

22. Vorrichtung nach Patentanspruch 21 , **dadurch gekennzeichnet, daß** die Reizelektroden (109) mit Halteplatte (110) in ein Halterklötzchen (116) eingesetzt sind, das an seiner den Reizelektroden abgewandten Seite eine weitgehend starre, jedoch elastische Halterlippe (117) hat, mit der es in Haltetaschen (118) eines Fixierungsbandes (119) eingesetzt wird, und daß das Fixierungsband mit dem Halterklötzchen (116) und den Reizelektroden (109, 110) mittels eines mit einer Hand bedienbaren Klemmverschlusses (120) stufenlos anpassbar und lösbar am zu untersuchenden Körperteil angesetzt wird.

23. Vorrichtung nach Patentanspruch 22, **dadurch gekennzeichnet, daß** die Halterungsvorrichtung für das Reizelektrodenpaar (109, 110) eine Halterlippe (117') für das Halterklötzchen (116) mit Reizelektroden hat, die breiter als das Fixierungsband (119) ist und an ihren beiden Längsenden Schlitze (117") aufweist, durch die das Fixierungsband (119) gezogen wird.

24. Vorrichtung nach Patentanspruch 22 oder 23, **dadurch gekennzeichnet, daß** die Halterklötzchen (116, 116') unterschiedliche Höhe zur Anpassung an die zu stimulierende Körperstelle haben.

25. Vorrichtung nach Patentanspruch 1 und einem oder mehreren der Patentansprüche 2 bis 24, **dadurch gekennzeichnet, daß** am Daumensteg (14; 24) ein Kraftmesser (237), beispielsweise als Biegebalkenmessbrücke, angebracht ist, der die Kraftwirkung auf den Daumensteg und ihre zeitliche Änderung und damit die Kraft am Muskel mißt.

26. Vorrichtung nach Patentanspruch 1 und einem oder mehreren der Patentansprüche 1 bis 24, **dadurch gekennzeichnet, daß** an der Hauptgeräteachse 1 ein Kraftmesser, beispielsweise als Torsionsmesser, angebracht ist, der die Kraft an der Hauptgeräteachse (1) zwischen der Kraftquelle (188, 189; 190) und dem Achsanschlag (125) und damit die vom Achsanschlag (125) mit den Rotations anschlägen (126,129) unterstützten Nachlasten vor deren Einwirkung auf den Muskel mißt.

27. Vorrichtung nach Patentanspruch 1 und einem oder mehreren der Patentansprüche 2 bis 24, **dadurch gekennzeichnet, daß** an der Linearachse (132) der Kraftmesser (237') angebracht ist, der die Kraft an dieser zwischen dem Linearmotor (191) bzw. den. Linearanschlägen (128, 131) und der Übersetzung/Kraftumlenkun.g (133; 143, 135, 136, 137) der Hauptgeräteachse (1) mißt.

28. Vorrichtung nach Patentanspruch 1 und einem oder mehreren der Patentansprüche 2 bis 24, **dadurch gekennzeichnet, daß** ein Potentiometer (239) an der Hauptgeräteachse (1) bzw ein Potentiometer (239') mit Übersetzungsseil (134') auf der Linearachse (132) angebracht sind, die die Stellung der Hauptgeräteachse (1) und die Länge der untersuchten Muskeln sowie die Geschwindigkeit ihrer Längenänderung messen.

29. Vorrichtung nach Patentanspruch 1 und einem oder mehreren der Patentansprüche 2 bis 28, **dadurch gekennzeichnet, daß** ein Beschleunigungsmesser (240) an der Daumenstegverschiebung (47; 93) des Daumenstegs (14; 24) die Beschleunigungen der Muskelkontraktion und der Muskelerschlaffung mißt.

30. Vorrichtung nach Patentanspruch 1 und einem oder mehreren der Patentansprüche 1 bis 29, **dadurch gekennzeichnet, daß** elektrischelektronische Sensoren (204, 204'; 205, 205') zur Aufnahme elektrischer Potentialschwankungen, der Hauttemperatur und von akustischen Signalen und Vibrationen vorhanden sind, die in direkten Kontakt mit den untersuchten Körperteilen gebracht werden.

31. Vorrichtung nach Patentanspruch 30, **dadurch gekennzeichnet, daß** die Sensoren zur Aufnahme elektrischer Patentialjchwankungen und der Hauttemperatur Elektrodenplättchen (204) sind, die in Elektrodennäpfchen (204') eingesetzt sind, welche zugleich zur Aufnahme von Kontaktmitteln dienen, und daß der Sensor zur Aufnahme von akustischen Signalen bzw. Vibrationen ein Vibrationssensor bzw. Mikrophon (205) mit Schallrohr (205') ist, die jeweils mit einer Verstärker- und Auswerteelektronik verbunden sind.

32. Vorrichtung nach Patentanspruch 1 und Patentanspruch 30 oder 31, **dadurch gekennzeichnet, daß** wenigstens ein Sensor (204, 204') sowie ein Sensor (205, 205') in einem Gehäuse (207) integriert und auf der unteren, zur Auflage auf den Körper bestimmten Ebene des Gehäuses angeordnet sind, und daß das Gehäuse (207) beweglich an der Verschienungsauflage (12) bzw. am Armauflagentisch (16) angeordnet ist (242 bis 246).

33. Vorrichtung nach Patentanspruch 1 und Patentanspruch 30 oder 31, **dadurch gekennzeichnet, daß** die Sensoren (208, 209) zur Aufnahme elektrischer Potentialschwankungen und der Hauttemperatur sowie der Sensor (211, 212) zur Aufnahme von akustischen Signalen bzw. Vibrationen mit Ausgleichsfedern (210, 213) im Sensorgehäuse (116) beaufschlagt sind, daß ferner das Gehäuse an seiner Unterseite, vorzugsweise auch auf einer dem untersuchten Körperteil abgewandten Randfläche selbstklebende Haftflächen (251, 252) hat und Bohrungen (253) von der Unterseite des Gehäuses (216) zu Schlauchanschlüssen an ein Vakuumgerät führen.

34. Vorrichtung nach Patentanspruch 1 und einem oder mehreren der Patentansprüche 1 bis 30, **dadurch gekennzeichnet, daß** bei der Untersuchung mit dieser eine Multisensoreinheit (217 bis 236) verwandt wird, in der neben Sensoren Vorrichtungen zur Darstellung, Speicherung und Verarbeitung der aufgenommenen Meßwerte integriert sind.

35. Vorrichtung nach Patentansprüchen 1 bis 3, **dadurch gekennzeichnet, daß** auf der Hauptgeräteachse 1 im Anschluß an die Drehmagnete (188, 189) eine in ihrer Kupplungskraft einstellbare Rutschkupplung (202) angesetzt ist.

36. Vorrichtung nach Patentansprüchen 1 bis 3, **dadurch gekennzeichnet, daß** der Daumenhebel (15) mit der Hauptgeräteachse (1) mittels eines Bolzens (203) mit Sollbruchstelle verbunden ist, wobei der Bolzen bei einem definierten Grenzwert bricht.

37. Vorrichtung nach Patentanspruch 1 und einem oder mehreren der Patentansprüche 2 bis 36, **dadurch gekennzeichnet, daß** auf dem Daumenhebel (15; 25) eine Meßskala (99) für die Verschiebeeinrichtung (47) angebracht ist.

38. Vorrichtung nach Patentansprüchen 1 und 7, **dadurch gekennzeichnet, daß** am Verschiebeklötzchen (52) des Armauflagentischs (16, 16') ein Linearmesser (100) zur Messung der Position des Auflagentischs angebracht ist.

39. Vorrichtung nach Patentansprüchen 1 und 7, **dadurch gekennzeichnet, daß** am Verschiebungsklötzchen (70) der Fingerstütze (19) ein Linearmesser (104) zur Messung der Position der Fingerstütze (19) angebracht ist.

40. Vorrichtung nach Patentansprüchen 1 und 7, **dadurch gekennzeichnet, daß** am Verschiebungsklötzchen (63) der Armstütze (18) ein Linearmesser (102) zur Messung der Position der Armstütze angebracht ist.

41. Vorrichtung nach Patentansprüchen 1 und 8, **dadurch gekennzeichnet, daß** an der Fingerspitzenstütze (20) ein Linearmesser (106) zur Messung der Position der Fingerspitzenstütze angebracht ist.

## Claims

1. A device to examine the motor system of the human or animal body through examination and determination of the function and performance capacity of the musculature, comprising a support and fixing device with a splinting support or with arm support table and with a limb fixation, in particular in the form of a thumb mould with thumb bridge and thumb lever, to insert, place and fix the body parts to be examined, such as for example the hand or the lower arm, comprising means for repeatable examination of the same body parts in the same fixed position, comprising a force generator to place load on the muscles, as well as stimulators to stimulate/contract the muscle, nerve or central nervous system and comprising mechanical and electric-electronic measuring equipment to measure force, acceleration and muscle lengths, characteristic in that the thumb lever (15,25), together with the thumb mould (13,23) and the thumb bridge (14,24), is positively connected to the main unit axle (1) arranged perpendicularly to the thumb lever (15,25) and that this main unit axle (1) is connected to the force generator in the form of one or two moving magnets (188,189) or an electromotor (190) or a linear drive (linear motor, lifting magnet) (191) resp. engages with the force generator (188,189;190;191), that furthermore - to determine the type of contraction and for automatic force and/or length limitation - the main unit axis (1) has an axle stop (125) between the connection to the thumb lever (15;25) and the force generator (188,189;190), which acts together with a front rotary stop (126) and a rear rotary stop (129), that the stimulation/contraction of muscle, nerve or central nervous system is actuated by the force generator (188,189;190;191) or through peripheral electrical stimulation by means of stimulators (109,110), that the force sensors (237,238) measure the dynamic effect on the thumb bridge (14;24) as well as their temporal change and the force at the main unit axle (1) between the force generator (188, 189; 190) and the axle stop (125), while the measurement of the acceleration of the muscle contraction is carried out via an accelerometer (240) on the thumb bridge slide (47;93) of the thumb bridge (14;24) and the potentiometer (239;239') measures the muscle length by measuring the position of the main unit axle (1) and that it comprises sensors for picking up electrical potential variations, skin temperature and acoustic signals and vibrations.

2. A device according to Patent Claim 1, characteristic in that the front rotary stop (126) acting together with the axle stop (125) is mounted on an adjustment wheel (143) and that its angle can be adjusted via this and the rear rotary stop (129) is mounted on an adjustment wheel (159) and its angle can also be adjusted via this.

3. A device according to Patent Claim 2, characteristic in that the adjustment wheel (143), via a bearing (144), is placed on a guiding rod (145), which grips the main unit axle (1) without pressure and is, on the side, distant to the stopping mechanism, equipped with a sprocket (146), into which a cogwheel (147) interlocks, which is driven by an adjustment motor (149) through an axle (148); the device is further characteristic in that the adjustment wheel (159), via a bearing (160), is fitted on a guiding rod (161), which grips the main unit axle (1) without pressure and the adjustment wheel (159) is, on the side, distant to the stopping mechanism, equipped with a sprocket (162), into which a cogwheel, (163) interlocks, which is driven by an adjustment motor (165), through an axle (164), where, on the adjustment motor (149) via an axle (174), a potentiometer (175) is placed for measuring the position of the front rotation stopping mechanism (126) and on the adjustment motor (165), via an axle (181), a potentiometer (182) is placed for measuring the position of the rear rotation stopping mechanism (129).

4. A device according to Patent Claim 1, characteristic in that, when using a power source (191) in form of a linear motor or lifting magnets between it and its connection to the main unit axle (1), a front axle stopping mechanism (127) is firmly placed on the linear axle (132) connecting to the power source, which forms the stopping mechanism for the front linear stopping mechanism (128), positioned diagonally to the linear axle (132), and that further a rear axle stopping mechanism (130) is firmly placed on the linear axle (132), which forms the stopping mechanism for the rear linear stopping mechanism (131), also positioned diagonally to the linear axle (132); hereby, the linear stopping mechanisms (128, 131) each consist of a bridge, which is guided by vertically positioned guiding rods (151, 167) and rests on these, via glide bearings (151, 166), which are adjustable parallel to the linear axle (132), where the adjustment of the linear stopping mechanism (128), occurs through a motor (158) via a spindle (154) and the adjustment of the linear stopping mechanism (131) occurs through a motor (173) via a spindle (169); a potentiometer (180) for measuring the position of the front linear stopping mechanism (128), is placed on the spindle (154) and a potentiometer (187) for measuring the position of the rear linear stopping mechanism (131), is placed on the spindle (169).

5. A device according to Patent Claim 1, characteristic in that, when using the rest-and securing element with the splint rest (12), it is fitted vertically on a diagonal casing wall (142) of the casing (140, 141, 142) of the device, and on that is an arm rest (3) with connecting hand rest (3'); arranged further to this, at a right angle to the splint rest (12) and the arm rest (3), are the back of the hand support (6) with the back of the hand cushion (7) and the adjustment bar (8), which are vertically adjustable through this adjustment bar (8) via an adapted slot (43') of the splint rest (12); further there is, also at a right angle to the arm rest (3), an arm rest (4) and on the hand rest (3') is a wrist support (10), horizontally adjustable via a carrier and adjustment track (44) and attached securely with a fastening screw (44'); a palm of the hand support (9), in connection with the wrist support (10), runs on the same level and attached to it is a finger support (11) for the fingers II-V, which is movable; the hand including the wrist is inserted and secured, so that the back of the hand support (6), including the back of the hand cushion (7) is moved vertically through an electrical motor (41) with an ammeter/ cut out (98) via a spindle (42), into which the adjustment bar (8) with the small adjustment block (43) meshes and that alternatively, the wrist support (10) on the adjustment track (44) is moved horizontally and locked in place with the adjusting screw (44'); the motor (41) and the spindle (42) are arranged on the side of the splint rest (12) away from the arm rest (3) through spindle tracks (42') and that the back of the hand support (6), with the connection bar (8), can be swivelled around a vertical axle via a swivel coupling (8') in the area where the spindle meshes; the finger support (11) is moved towards the index finger with the aid of a small adjustment block (46) on an adjustment track (45) and locked in place with the adjusting screws (46').

6. A device according to one or more of the Patent Claims 1 to 5, characteristic in that the splint rest (12) is fitted horizontally and parallel to the dividing wall (142) of the device casing and is movable/adjustable on one or more guide tracks (40), which are fitted to the dividing wall (142) with mountings (40) and arranged as spindles by means of an electrical motor (38).

7. A device according to Patent Claim 1, characteristic in that, with the rest- and securing element and the arm rest table (16, 16'), the table is horizontally movable on tracks (58), parallel to the diagonal casing wall (142), above which, the arm rest table (16) is guided by a spindle (53) and powered by a motor (56), which is installed below the arm rest table on the diagonal casing wall (142) via a small connection block (52), which is firmly attached to the underneath of the arm rest table (16) and guided in a spindle (53) with spindle tracks (54) on the underneath of the arm rest table and is being moved towards the spindle (53) by a transmission belt (55); the device is characteristic further, in that an arm rest (17) is fitted centrally for placing the arm and hand for both sides, on which an arm clamp cushion (21), with fastening clamp (85) and clamp bracket (82), is fitted to its top side and, a distance apart from it, a palm of the hand clamp cushion (22) with fastening clamp (90) and clamp bracket (88), which can - with screws (84, 89'), in rows with the holes (83, 89) - each be shifted lengthways of the arm rest (17), through which the arm clamp cushion (21) and the palm of the hand cushion (22) can also be shifted by 180° in order to secure the respective inserted arm and hand; the arm clamp cushion is adjustable in height by means of a guide bar (86) with a locking mechanism (87), and the palm of the hand clamp cushion (22), by means of a guide bar (91) with a locking mechanism (92), for the adjustment of the palm of the hand and arm; further to this, there is, on both sides of the arm rest (17) on the arm rest table (16), a movable finger support (19), which has a swivel coupling (68) with swivel coupling pin (68'), through which, together with an elongated hole (69) in the arm rest table (16), is a connection to a small adjustment block (70), which leads, through a spindle (71) with counter-rotating threads and a motor (74) with transmission belt (73) for the spindle, the finger supports (19) are running opposite towards the splint to the respective inserted hand, where the spindle (71), with spindle tracks (72), as well as the motor (74), are attached to the underneath of the arm rest table (16); further to this, there are, relating to the finger supports (19), intended for the support of the outer side of the arm in the area of the arm clamp cushion (21), on both sides of the arm rest (17), arm supports (18) with swivel coupling (61), the swivel coupling pin (61'), the small adjustment block (63), the spindle (64) with spindle tracks (65) and the motor (67) with transmission belt (66), which together will move towards the inserted arm.

8. A device according to Patent Claim 7, characteristic in that, to support the finger tips on the rest- and securing element with arm rest table (16), a movable support (20) is placed on that table, with a small adjustment block (75), guided into a spindle (76) with spindle tracks (77) on the arm rest table, which is moved by a motor (80) with a transmission belt (78) and moves the support (20) on telescopic tracks (81) towards the finger tips of the hand to be examined hand which is secured in place.

9. A device according to one or more of the Patent Claims 1 to 7, characteristic in that, the rest- and securing elements (3 to 12) or (16 to 22) can be interchanged, and be used as separate modules of the device, or other splint rest elements, where they should preferably be separated from the remaining devices by a double casing wall (142).

10. A device according to one or more of the Patent Claims 1 to 8, characteristic in that, for the finger to be examined or thumb of the inserted hand, a thumb mould (13) with the thumb bridge (14) or else the thumb mould (23) with the thumb bridge (24), are available, whereby the thumb bridge (14; 24) connects to the horizontally placed thumb lever (15; 25) and is horizontally movable by means of an adjustment device (47; 93) with locking mechanism (47', 94).

11. A device according to one or more of the Patent Claims 1 to 10, characteristic in that, positioned on the top end of the main unit axle (1), is a light beamer, preferably a laser direction-finding beamer (37) with an aperture (37'), whose direction-finding beam is pointed to the body part to be examined, for example, the hand, and is aligned with its movement axle before securing.

12. A device according to one or more of the Patent Claims 1 to 10, characteristic in that, instead of or additionally to, the laser direction-finding beamer (37), as in Fig. 10, a laser direction-finding beamer (48) is arranged at the end of a swivel arm (49), which with its other end can swivel and is attached to the track (58) of the arm rest table (16) and will be swivelled over the body part resting in it and being examined, in which the laser direction-finding beamer (48) and its direction-finding beam, are aligned, whilst in the position that it has been swivelled into, with the main unit axle (1) and matched up with it, whereby the movement axle of the body part to be examined, is brought into alignment with the direction-finding beam, before securing.

13. A device according to Patent Claim 1 and one or more of the Patent Claims 2, 3, 4, 10, 11 and 12, characteristic in that, with the rest-and securing element and arm rest table (16, 16'), the table is horizontally movable in the tracks (58), parallel to the diagonal casing wall (142); a cast (29), made from synthetic or other material, is used as a negative form for the resting and securing of the hand to be examined, and will be inserted into the corresponding drill holes (28') of the arm rest table (16), by means of the imbedded spacing pins (28), which protrude on the underneath of the cast (29) in such a way, that the movement axle of the hand will be aligned with the main unit axle of the device and the cast (29) will have a cavity cut out (30) in the area of the thumb and the thumb muscle, for the thumb mould (23) with a thumb bridge (24), so that the thumb can be positioned and will have a connection to the thumb lever (25) and the main unit axle (1).

14. A device according to Patent Claim 13, characteristic in that, for the making of the cast (29), a mould (27) is used, with spacing pins (28) at the bottom of it, which can be removed; a laser direction-finding beamer, with a swivel arm (96), is positioned on one of the top outer rims of the mould (27), whereby for the making of the cast (29), the hand is placed with the back of the hand onto the spacing pins (28), then the laser direction-finding beamer (96) is swivelled over it and its direction-finding beam aligned with the movement axle of the hand, as the mould (27) with the hand in it, will be filled in with suitable plastic or other hardening pouring substance.

15. A device according to Patent Claims 1 to 4, characteristic in that they have a rest plate (32) for resting and securing those body parts with big joints - for example the ankle joint - which has a fastening ring (33) with a locking mechanism for this ring (34) for firmly fastening onto the main unit axle (1), whereby a fastener (36) is additionally attached to the rest plate (32), as well as a measuring scale (108) at the elongated hole (97) for measuring the position of the body part being examined.

16. A device according to Patent Claim 1 and one or more of the Patent Claims 2, 3, 5 to 15, characteristic in that a rotation magnet (188) with constant or constantly increasing indicator line is used as a power source.

17. A device according to Patent Claim 1 and one or more of the Patent Claims 2, 3, 5 to 15, characteristic in that two rotation magnets (188, 189) are used as the power source, preferably with a constant total or a constantly increasing indicator line, which are switched, one after the other, and are being rotated around the inner part connected to the main unit axle (1), whilst a sprocket (194) is positioned between the rotation magnets (188, 189) - which is connected to a motor (199) through a rack track (195) with a track bearing (196), a cog wheel (197) and a transmission belt (198) - which adjusts the rotation magnets (188, 189); a potentiometer (210) is fitted to the motor (199) for measuring the position of the rotation magnets (188, 189).

18. A device according to Patent Claim 1 and one or more of the Patent Claims 2, 3, 5 to 15, characteristic in that an electric motor (190) is used as a power source, preferably in the form of a Hall motor.

19. A device according to Patent Claim 1 and one or more of the Patent Claims 4 to 15, characteristic in that a linear drive (191), in the form of a linear motor or lifting magnet, is used as a power source, which transmits power through the linear axle (132) and a transmission/ power diversion (133, 134, 135, 136, 137) onto the main unit axle (1), positioned at a right angle to the linear axle; a transmission wheel (133) sits firmly on the main unit axle (1) in the area of the linear axle and with a transmission cord (134), half of which is running on top of the transmission wheel (133) to the lower cord clip (136) on the linear axle (132) and will be fixed in place, and the other half is running under the transmission wheel (133) to the upper cord clip (136) on the linear axle (132) and will be fixed in place; attached on the side opposite the transmission cord (134) of the linear axle (132), is a counter-moving cord (135) with clamps (136'), which are themselves connected to the linear axle (132) via clamp bridges (137), to avoid one-sided strain and bending of the linear axle (132); the linear axle is resting on a guiding bar (139), with glide bearing (138) and with its end opposite the power source (191).

20. A device according to Patent Claim 1 and one or more of the Patent Claims 2 to 19, characteristic in that the stimulation of the motor system of the person being examined, takes place in the form of voluntary triggers, central and peripheral magnetic stimulation, reflex stimulation - preferably through changes of the muscle length through the power source or as peripheral electrical stimulation through stimulants with stimulation electrodes.

21. A device according to Patent Claim 20, characteristic in that the stimulation electrodes are used as a pair of twin-stimulation electrodes (109, 110), each pair having mounting plates (110), with two stimulation electrodes inserted and each pair of electrodes is connected to a small switch box (114) via a cable and plug (112,113), which has a single switch for each pair of stimulation electrodes and a main switch (115'); the pairs of stimulation electrodes are connected to the control electronics of the control unit device through a cable and plug (112', 113').

22. A device according to Patent Claim 21, characteristic in that the stimulation electrodes (109), with the mounting plate (110), are placed into a small mounting block (116), which has - on the off side of the stimulation electrodes - an elastic, but mostly rigid mounting lip (117), with which it sits in the mounting pockets (118) of a fastener (119); the fastener with the small mounting block (116) and the stimulation electrodes (109, 110), will be placed on the body part to be examined by means of a clamp fastener (120), which can be operated with one hand and is smoothly adjustable and removable.

23. A device according to Patent Claim 22, characteristic in that the mounting device for the pair of stimulation electrodes (109, 110) has a mounting lip (117') - for the small mounting block (116), with stimulation electrodes - which is wider than the fastener (119) and has slits (117") on its two ends, through which the fastener (119) is being pulled through.

24. A device according to Patent Claim 22 or 23, characteristic in that the small mounting blocks (116, 166') have different heights, so that they can be adjusted to the body area to be stimulated.

25. A device according to Patent Claim 1 and one or more of the Patent Claims 2 to 24, characteristic in that a dynamometer (237) is attached to the thumb bridge (14; 24), for example as a bending beam measuring gauge, which measures the power exerted on the thumb bridge and its time changes, and therefore measures the strength of the muscle.

26. A device according to Patent Claim 1 and one or more of the Patent Claims to 24, characteristic in that a dynamometer is attached to the main unit axle (1), for example as a torsion meter, which measures the strength on the main unit axle (1) between the power source (188, 189, 190) and the axle stopping mechanism (125), and therefore measures the residual weights supported by the axle stopping mechanism (125) and the rotation stopping mechanism (126, 129) before they affect the muscle.

27. A device according to Patent Claim 1 and one or more of the Patent Claims 2 to 24, characteristic in that the dynamometer (237') is attached to the linear axle (132), which measures the power between the linear motor (191) or the linear stopping mechanism (128, 131) and the transmission/ power diversion (133, 143, 135, 136, 137) of the main unit axle (1).

28. A device according to Patent Claim 1 and one or more of the Patent Claims 2 to 24, characteristic in that a potentiometer (239) is attached to the main unit axle (1), or a potentiometer (239') with transmission cord (134') to the linear axle (132), which measures the position of the main unit axle (1) and the length of the examined muscles, as well as the speed of the muscles' change in length.

29. A device according to Patent Claim 1 and one or more of the Patent Claims 2 to 28, characteristic in that an acceleration meter (240) on the thumb bridge adjustment (47; 93) of the thumb bridge (14; 24), measures the acceleration of the muscle contraction and the muscle relaxation.

30. A device according to Patent Claim 1 and one or more of the Patent Claims 2 to 29, characteristic in that electrical/electronic sensors (204, 204', 205, 205') are included, for the recording of electrical potential fluctuations, the skin temperature and acoustic signals and vibrations, which are being brought into direct contact with the body parts being examined.

31. A device according to Patent Claim 30, characteristic in that the sensors for the recording of electrical potential fluctuations, and of the skin temperature, are in the form of small electrode plates (204), which are inserted into the recesses for the small electrode (204'), which are used at the same time hold contact substances; that the sensor for the recording of acoustic signals or vibrations is a vibration sensor or microphone (205) with a sound tube (205'), which are each connected to an amplifier and analysis electronics.

32. A device according to Patent Claim 1 and Patent Claim 30 or 31, characteristic in that at least one sensor (204, 204'), as well as a sensor (205, 205') in the casing (207), is integrated and positioned on the bottom level of the casing - which is intended for resting on the body of the person to being examined - and that the casing (207) is positioned, so that it is movable on the splint rest (12) or on the arm rest table (16). (242 to 246)

33. A device according to Patent Claim 1 and Patent Claim 30 or 31, characteristic in that the sensors (208, 209) for the recording of electrical potential fluctuations and the skin temperature, as well as sensors (211, 212) for the recording of acoustic signals or vibrations, are equipped with differential springs (210, 213) in the sensor casing (116); further that the casing has self-adhesive patches (251, 252) on its underside side and preferably also on one of the rim areas of the side facing away from the body part being examined, and that drill holes (253) from the underside of the casing (216) lead to pipe connectors on a vacuum unit.

34. A device according to Patent Claim 1 and one or more of the Patent Claims 1 to 30, characteristic in that during the examination, a multi-sensor unit (217 to 236) is being used, in which, apart from sensors, there are also integrated devices for the display, storage and processing of the recorded measuring results.

35. A device according to Patent Claims 1 to 3, characteristic in that a slip clutch mechanism (202), which is adjustable in its working area, is attached to the main unit axle (1) in connection with the rotation magnets (188, 189).

36. A device according to Patent Claims 1 to 3, characteristic in that the thumb lever (15) is connected to the main unit axle (1) by means of a pin (203) with a predefined breaking point, where the pin breaks when a defined limited of the power source is exceeded.

37. A device according to Patent Claim 1 and one or more of the Patent Claims 2 to 36, characteristic in that a measuring scale (99) for the adjustment unit (47) is connected to the thumb lever (15; 25).

38. A device according to Patent Claims 1 to 7, characteristic in that a linear meter (100) for measuring the position of the armrest table (16, 16'); is attached to the small adjustment block (52) of the armrest table (16, 16').

39. A device according to Patent Claims 1 to 7, characteristic in that a linear meter (104) for measuring the position of the finger support (19) is attached to the small adjustment block (70).

40. A device according to Patent Claims 1 to 7, characteristic in that a linear meter (102) for measuring the position of the arm support (18), is attached to the small adjustment block (63) of the arm support (18).

41. A device according to Patent Claims 1 to 8, characteristic in that a linear meter (106) for measuring the position of the fingertip support, is attached to the fingertip support (20).

## Revendications

1. Dispositif d'examen du système moteur du corps humain ou animal par examen et détermination du fonctionnement et de la puissance des muscles, comprenant un système d'appui et de fixation avec une cheville de maintien ou une table d'appui pour les bras ainsi qu'une attache pour les membres, notamment sous forme de gaine pour le pouce comportant une tige et un levier, pour poser, maintenir et fixer la partie du corps devant être examinée, comme par exemple la main ou l'avant-bras, avec des moyens d'examen répété de ces même parties du corps dans la même position fixe, avec une source d'énergie pour contraindre les muscles et des stimulateurs pour stimuler/contracter les muscles, les nerfs ou le système nerveux central et avec des instruments de mesure mécaniques et électro-électroniques pour mesurer la force, l'accélération et les longueurs des muscles,
**caractérisé en ce que** le levier du pouce (15, 25), avec la gaine (13, 23) et la tige (14, 24), est relié par adhérence à l'axe principal (1) disposé verticalement par rapport au levier du pouce (15, 25) et que cet axe principal (1) est raccordé avec la source d'énergie sous forme d'un ou de deux aimants mobiles (188, 189) ou d'un moteur électrique (190) ou d'un actionnement linéaire (moteur linéaire,
électroaimant de levage) (191) ou bien est engrené dans la source d'énergie (188, 189, 190; 191), **en ce qu'**en plus, pour déterminer le type de contraction et pour limiter automatiquement l'énergie et/ou la longueur, l'axe principal (1) du dispositif présente entre la jonction du levier du pouce (15; 25) et de la source d'énergie (188, 189; 190) un arrêtoir de l'axe (125) fonctionnant par action conjointe d'un arrêtoir de rotation avant (126) et un arrêtoir de rotation arrière (129), **en ce que** la stimulation/contraction des muscles, nerfs ou du système nerveux central est provoquée par la source d'énergie (188, 189; 190; 191) ou par stimulation électrique périphérique au moyen de stimulateurs (109, 110), **en ce que** les dynamomètres (237, 238) mesurent l'effet dynamique sur la tige du pouce (14 24) ainsi que sa variations temporaire et l'énergie sur l'axe principal (1) du dispositif entre la source d'énergie (188, 189; 190) et l'arrêtoir (125) de l' axe, tandis que l'accélération de la contraction des muscles est mesurée par un accéléromètre (240) sur le déplacement (47; 93) de la tige du pouce (14; 24) et le potentiomètre (239; 239') mesure la longueur des muscles en mesurant la position de l'axe principal (1) du dispositif et **en ce qu'**il y a des capteurs pour enregistrer les variations de potentiel électriques, la température de la peau et les signaux acoustiques et les vibrations.

2. Dispositif selon la revendication 1, **caractérisé en ce que** l'arrêtoir de rotation avant (126) fonctionnant en concours avec l'arrêtoir de l'axe (125) est placé sur une roue réglable (143), son angle étant ajustable au moyen de cette roue, et **en ce que** l'arrêtoir de rotation arrière (129) est placé sur une roue réglable (159) et son angle aussi ajustable au moyen de cette roue.

3. Dispositif selon la revendication 2, **caractérisé en ce que** la roue réglable (143) est logée au moyen d'un palier (144) sur un tube conducteur (145) enveloppant l'axe principal (1) du dispositif sans liaison par adhérence et, du côté le plus éloigné de l'arrêtoir, est en outre munie d'une jante dentée (146) dans laquelle est engrenée une roue dentée (147), elle-même actionnée par l'intermédiaire de l'axe (148) par un servomoteur (149), **caractérisé** de surcroît **en ce que** la roue réglable (159) est logée au moyen d'un palier (160) sur le tube conducteur (161) enveloppant l'axe principal (1) du dispositif sans liaison par adhérence, et la roue réglable (159), du côté le plus éloigné de arrêtoir, est munie de la jante dentée (162), dans laquelle est engrenée une roue dentée (163) actionnée par l'intermédiaire d'un axe (164) par un servomoteur (165), un potentiomètre (175) étant fixé sur le servomoteur (149) au-dessus d'un axe (174) pour mesurer la position de l'arrêtoir de rotation avant (126) et un potentiomètre (182) étant fixé sur le servomoteur (165) au-dessus d'un axe (181) pour mesurer la position de l'arrêtoir de rotation arrière (129).

4. Dispositif selon la revendication 1, **caractérisé en ce qu'**en utilisant la source d'énergie (191) sous forme d'un moteur linéaire ou d'électroaimants de levage, est solidement disposé entre elle et sa jonction avec l'axe principal (1) du dispositif un arrêtoir avant (127) sur l'axe linéaire (132) à la suite de la source d'énergie, cet arrêtoir constituant l'arrêt pour l'arrêtoir linéaire avant (128) placé transversalement par rapport à l'axe linéaire (132), et **en ce qu'**en outre un arrêtoir arrière (130) est solidement disposé sur l'axe linéaire (132), constituant l'arrêt pour l'arrêtoir linéaire arrière (131) également placé transversalement par rapport à l'axe linéaire (132), les arrêtoirs linéaires (128, 131) étant respectivement formés par une tige respectivement guidée par des tiges de guidage (151, 167) disposées verticalement par rapport à eux et déplaçables sur elles au moyen de paliers à glissement (151, 166) parallèles à l'axe linéaire (132), et le déplacement de l'arrêtoir linéaire (128) se faisant par un moteur (158) par l'intermédiaire d'une broche (154) et le déplacement de l'arrêtoir linéaire (131) se faisant par un moteur (173) par l'intermédiaire d'une broche (169), et **en ce qu'**un potentiomètre (180) est installé sur la broche (154) pour mesurer la position de l'arrêtoir linéaire avant (128) et un potentiomètre (187) est installé sur la broche (169) pour mesurer la position de l'arrêtoir linéaire arrière (131).

5. Dispositif selon la revendication 1, **caractérisé en ce que** dans le système d'appui et de fixation avec cheville de maintien (12), celle-ci est disposée verticalement sur une paroi transversale (142) du boîtier (140, 141, 142) du dispositif et qu'un appui-bras (3) suivi d'un appui-main (3') sont installés dessus, **en ce qu'**en outre, en angle droit par rapport à la cheville de maintien (12) et à l'appui-bras (3), sont disposés les supports pour le dos de la main (6) garni d'un rembourrage (7) pour le dos de la main et d'une tige de déplacement (8) qui sont déplaçables dans le sens vertical par l'intermédiaire de cette tige de déplacement (8) dans un creux adapté (43') dans la cheville de maintien (12), **en ce qu'**il y a de surcroît un appui-bras (4) également disposé en angle droit par rapport à l'appui- bras (3) et, sur l'appui-main (3'), est fixé un support pour le poignet (10) horizontalement déplaçable au moyen d'une tige de support et de déplacement (44) et blocable avec une vis de fixation (44'), **en ce qu'**en outre un support pour la paume de la main (9) est disposé à la suite du support pour le poignet (10) et au même niveau que celui-ci, ainsi qu'un support (11) pour les doigts II - V mobile, la main mise en place ainsi que le poignet étant fixés de manière que le support pour le dos de la main (6) avec le rembourrage (7) soit déplacé verticalement au moyen d'un moteur électrique (41) avec ampèremètre/interrupteur de courant (98) par l'intermédiaire d'une broche (42), dans laquelle est engrenée la tige de déplacement (8) à l'aide d'un petit bloc de déplacement (43), et **en ce que**, d'autre part, le support pour le poignet (10) est déplacé horizontalement sur la tige de déplacement (44) et bloqué au moyen de la vis d'arrêt (44'), le moteur (41) et la broche (42) étant disposés sur le côté de la cheville de maintien (12) opposé à l'appui-bras (3) à l'aide de glissières (42'), et **en ce que** le support pour dos de la main (6) est orientable avec la tige de jonction (8) par l'intermédiaire d'une charnière (8') dans la zone d'engrènement dans la broche autour d'un axe vertical et que le support pour les doigts (11) est approché de l'index à l'aide d'un petit bloc cale de déplacement (46) sur une glissière de déplacement (45) et bloqué avec la vis d'arrêt (46').

6. Dispositif selon l'une ou plusieurs des revendications 1 à 5, **caractérisé en ce que** la cheville de maintien (12) est déplaçable horizontalement et parallèlement à la paroi intermédiaire (142) du boîtier du dispositif sur une ou plusieurs tiges de guidage (40), fixées à la paroi intermédiaire avec des attaches (40') et se présentant sous forme de broches, au moyen d'un moteur électrique (38).

7. Dispositif selon la revendication 1, **caractérisé en ce que** dans le système d'appui et de fixation avec table de support pour bras (16, 16'), celle-ci est placée dans des glissières (58) parallèlement et à la paroi transversale du boîtier (142) au-dessus d'elle et est déplaçable à l'horizontale, la table de support pour bras (16) étant guidée par un moteur (56) monté en dessous de la table de support pour bras sur la paroi transversale du boîtier (142) par l'intermédiaire d'un petit bloc de jonction (52) solidement fixé sur le dessous de la table de support pour bras (16) et dans une broche (53) avec des glissières (54) sur le dessous de la table de support pour bras et déplacée par l'intermédiaire de courroies de transmission (55) vers la broche (53), **caractérisé en outre en ce qu'**est placé au milieu un appui-bras (17) pour maintenir des deux côtés le bras posé ainsi que la main, sur le côté supérieur duquel est fixé un coussin de serrage du bras (21) avec des boucles de retenue (85) et boucles d'attache (82) et, à un écart de celui-ci, un coussin de serrage de la main (22) avec des boucles de retenue (90) et des boucles d'attache (88), qui sont respectivement déplaçables dans le sens de la longueur de l'appui-bras (17) avec des vis (84, 89') disposées en rangées de trous (83, 89), ce qui fait que le coussin de serrage du bras (21) et le coussin de serrage de la main (22) sont aussi respectivement déplaçables de 180° par rapport, pour fixer le bras et la main respectivement maintenus, le coussin de serrage du bras étant déplaçable en hauteur au moyen d'une barre de déplacement (86) dotée d'un système d'arrêt (87) et le coussin de serrage de la main (22) étant déplaçable en hauteur au moyen d'une barre de déplacement (91) dotée d'un système d'arrêt (92), pour les adapter à la main et au bras, **caractérisé** de surcroît **en ce que**, des deux côtés de l'appui-bras (127), est respectivement placé sur la table d'appui du bras (16) un support pour les doigts (19) mobile ayant une charnière (68) avec une tige (68') et relié par elle et un trou longitudinal (69) percé dans la table d'appui du bras (16) à un petit bloc de déplacement (70) conduisant, par une broche (71) avec des filets opposés et par un moteur (74) avec des courroies de translation (73) pour la broche, les supports pour les doigts (19) vers la main respectivement maintenue, dans le sens opposé à la cheville, et la broche (71) à coulisses (72) ainsi que le moteur (74) étant fixés sur le dessous de la table d'appui du bras (16) et, par analogie avec les supports pour les doigts (19), pour soutenir la partie externe du bras dans la zone du coussin de serrage du bras (21) des deux côtés de l'appui-bras (17), des supports pour les bras (18) avec la charnière (61) et sa tige (61'), le petit bloc de déplacement (63), la broche (64) à glissières (65) et le moteur (67) à courroies de translation (66) étant en outre prévus et approchés du bras posé.

8. Dispositif selon la revendication 7, **caractérisé en ce que** pour soutenir les bouts des doigts dans le dispositif d'appui et de fixation avec table de support pour bras (16), un support (20) mobile (20) est placé sur celle-ci avec un petit bloc de déplacement (75) guidé sur la table de support pour bras dans une broche (76) à glissières (77) mues par un moteur (80) à courroies de translation (78) et approchant le support (20), avec des glissières télescopiques (81), des bouts des doigts de la main examinée pour les fixer.

9. Dispositif selon l'une ou plusieurs des revendications 1 à 7, **caractérisé en ce que** les dispositifs d'appui et de fixation (3 à 12) ou (16 à 22) peuvent, en tant que modules détachables du dispositif, être échangés entre eux ou contre d'autres systèmes de chevilles, mais en étant de préférence séparés du reste du dispositif par la double paroi du boîtier (142).

10. Dispositif selon l'une ou plusieurs des revendications 1 à 8, **caractérisé en ce que** pour le doigt ou le pouce à examiner de la main insérée, il y a une gaine (13) avec une tige (14) ou une gaine (23) avec une tige (24), cette tige (14; 24) étant engrenée dans le levier (15; 25) du pouce placé horizontalement et étant déplaçable horizontalement dessus à l'aide du dispositif de déplacement (47; 93) avec les arrêtoirs (47', 94).

11. Dispositif selon l'une ou plusieurs des revendications 1 à 10, **caractérisé en ce qu'**à l'extrémité supérieure de l'axe principal (1) du dispositif est fixé un projecteur à faisceau lumineux, de préférence un projecteur à faisceau radiogoniométrique laser (37) avec écran (37'), dont le faisceau radiogoniométrique est dirigé sur la partie du corps devant être examinée, la main par exemple, et coïncide avec son axe de mouvement avant sa fixation.

12. Dispositif selon l'une ou plusieurs des revendications 1 à 10, **caractérisé en ce qu'**à la place ou en plus du projecteur à faisceau radiogoniométrique laser (37) selon la fig. 10, un projecteur à faisceau radiogoniométrique laser (48) est disposé à l'extrémité d'un bras articulé (49) dont l'autre extrémité est mobile et fixée sur la coulisse (58) de la table de support pour le bras (16) et pivote au-dessus de la partie du corps maintenue devant être examinée, le projecteur à faisceau radiogoniométrique laser et son faisceau radiogoniométrique se trouvant, en position pivotante, dans le prolongement imaginé de l'axe principal (1) du dispositif et concordant avec lui et l'axe de mouvement de la partie du corps à examiner coïncidant avant sa fixation avec le faisceau radiogoniométrique.

13. Dispositif selon la revendication 1 et une ou plusieurs des revendications 2, 3, 4, 10, 11 et 12, **caractérisé en ce que** dans le système d'appui et de fixation avec table de support pour bras (16, 16') placée sur les coulisses (58) parallèlement à la paroi transversale du boîtier (142) et déplaçable horizontalement, un moule (29) en matière synthétique ou en une autre matière est disposé en tant que matrice négative pour maintenir et fixer la main à examiner, au moyen d'entretoises (28) moulées dépassant du dessous du moule (29), dans des trous (28') adaptés percés dans la table de support pour bras (16), de manière telle que l'axe de mouvement de la main coïncide avec l'axe principal du dispositif et le moule (29) présente dans la zone du pouce et du muscle du pouce une découpe (30) pour la gaine du pouce (23) avec la tige (24), pour poser le pouce et le relier au levier (25) et à l'axe principal (1) du dispositif.

14. Dispositif selon la revendication 13,
**caractérisé en ce qu'**une valve (27) avec des entretoises (28) démontables enfoncées dans son fond, sert à fabriquer le moule (29) et que sur un des bords extérieurs supérieurs de la valve (27) est placé un projecteur à faisceau radiogoniométrique laser pourvu d'un bras articulé (96) et que, pour la fabrication du moule (29), le dos de la main est posé sur les entretoises (28), le projecteur à faisceau radiogoniométrique laser (96) est dirigé dessus, son faisceau radiogoniométrique devant coïncider avec l'axe de mouvement de la main, et la valve (27), avec la main, est modelée avec une matière synthétique appropriée ou toute autre matière fondue durcie.

15. Dispositif selon l'une des revendications 1 à 4,
**caractérisé en ce qu'**il comporte une plaque de support (32) pour maintenir et fixer les parties du corps ayant de grosses articulations, comme par exemple le jarret, au-dessous de laquelle est installé un anneau de support (33) avec un arrêtoir (34) pour une fixation par adhérence à l'axe principal (1) du dispositif, et **en ce que** la plaque de support (32) possède, sur une partie de sa longueur, un trou longitudinal (97), dans lequel un bloc de soutien (35) mobile est disposé sur le dessus de la plaque de support et peut être bloqué avec le vissage (35'), pour soutenir et fixer la partie du corps avec son axe de mouvement sur l'axe principal (1) du dispositif, une bande de fixation (36) étant en plus fixée sur la plaque de support (32) et une échelle de mesure (108) étant placée sur le trou longitudinal (97) pour mesurer la position de la partie du corps examinée.

16. Dispositif selon la revendication 1 et une ou plusieurs des revendications 2, 3, 5 à 15,
**caractérisé en ce qu'**un aimant mobile (188) avec une ligne caractéristique constante ou constamment croissante sert de source d'énergie.

17. Dispositif selon la revendication 1 et une ou plusieurs des revendications 2, 3, 5 à 15,
**caractérisé en ce que** deux aimants mobiles (188, 189), de préférence avec une ligne caractéristique en somme constante ou constamment croissante, servent de source d'énergie, ces aimants étant montés l'un après l'autre et tournant autour de la partie intérieure reliée à l'axe principal (1) du dispositif, une jante dentée (194) étant disposée entre les aimants mobiles (188, 189) et reliée à un moteur (199) par une crémaillère (195) dotée d'un palier (196), une roue dentée (197) et une courroie de translation (198) et qui ajuste de cette manière les aimants mobiles (188, 189), et **en ce qu'**un potentiomètre (201) est disposé sur le moteur (199) pour mesurer la position des aimants mobiles (188, 189).

18. Dispositif selon la revendication 1 et une ou plusieurs des revendications 2, 3, 5 à 15,
**caractérisé en ce qu'**un moteur électrique (190), de préférence un moteur de Hall, sert de source d'énergie.

19. Dispositif selon la revendication 1 et une ou plusieurs des revendications 4 à 15,
**caractérisé en ce qu'**un actionnement linéaire (191) sous forme de moteur linéaire ou d'électroaimant de levage sert de source d'énergie et transmet l'énergie par l'axe linéaire (132) et une transmission/déviation (133, 134, 135, 136, 137) à l'axe principal (1) du dispositif placé en angle droit par rapport à l'axe linéaire, une roue de translation (133) étant solidement fixée sur l'axe principal (1) du dispositif, dans la zone de l'axe linéaire (132), avec un câble de translation (134) dont une moitié passe en haut par la roue de translation (133) en direction d'une pince de câble (136) inférieure sur l'axe linéaire (132) et est fixée et dont l'autre moitié passe en bas par la roue de translation (133) en direction d'une pince de câble (136) supérieure sur l'axe linéaire (132) et est fixée, et **en ce que** sur le côté de l'axe linéaire (132) opposé au câble de translation (134) est installé un câble de contre-traction (135) garni de pinces (136'), elles-mêmes fixées sur l'axe linéaire (132) par des tiges de serrage (137), pour empêcher la contrainte unilatérale et la courbure de l'axe linéaire (132), et l'axe linéaire étant logé, à son extrémité opposé à la source d'énergie (191), dans une tige de guidage (139) avec un palier à glissement (138).

20. Dispositif selon la revendication 1 et une ou plusieurs des revendications 2 à 19,
**caractérisé en ce que** la stimulation du système moteur se fait sous forme de déclenchement volontaire, de stimulation magnétique centrale ou périphérique, de stimulation de réflexes, de préférence par modification de la longueur des muscles avec la source d'énergie ou en tant que stimulation électrique périphérique par stimulateurs avec des électrodes stimulantes.

21. Dispositif selon la revendication 20,
**caractérisé en ce que** les électrodes stimulantes se présentent sous forme d'une paire d'électrodes stimulantes jumelles (109, 110), dont deux électrodes stimulantes (109) sont respectivement placées dans une plaque de support et de retenue (110) et chaque paire d'électrodes est raccordée par un câble et une fiche (112, 113) à un coffret de commande possédant pour chaque paire d'électrodes stimulantes un commutateur individuel (115) et un commutateur commun (115'), et **en ce que** les paires d'électrodes stimulantes sont reliées à l'équipement électronique de commande du dispositif de commande par un câble et une fiche (112', 113').

22. Dispositif selon la revendication 21,
**caractérisé en ce que** les électrodes stimulantes (109) avec la plaque de support (110) sont fixées dans un petit bloc de retenue (116) ayant sur son côté opposé aux électrodes stimulantes une lèvre de retenue (117) amplement rigide mais élastique, avec laquelle il est fixé dans des poches de maintien (118) d'une bande de fixation (119) et **en ce que** la bande de fixation avec le petit bloc de retenue (116) et les électrodes stimulantes (109, 110) est fixée à la partie du corps devant être examinée, à l'aide d'un blocage à pinces (12) maniable avec une main, de manière adaptable en continu et détachable.

23. Dispositif selon la revendication 22,
**caractérisé en ce que** le dispositif d'attache de la paire d'électrodes stimulantes (109, 110) a une lèvre de retenue (117') pour le petit bloc de retenue (115) avec des électrodes stimulantes, celle-ci étant plus large que la bande de fixation (119) et présentant sur ses deux extrémités longitudinales des fentes (117") à travers desquelles on fait passer la bande de fixation (119).

24. Dispositif selon l'une des revendications 22 ou 23, **caractérisé en ce que** les petits blocs de retenue (116, 116') ont différentes hauteurs pour l'adaptation à la partie du corps devant être stimulée.

25. Dispositif selon la revendication 1 et l'une ou plusieurs des revendications 2 à 24,
**caractérisé en ce que** sur la tige pour le pouce (14; 24) est placé un dynamomètre (237), par exemple sous forme d'ohmmètre à pont à barre souple, mesurant l'effet dynamique sur la tige pour le pouce et sa variation temporaire et donc la force du muscle.

26. Dispositif selon la revendication 1 et l'une ou plusieurs des revendications 1 à 24,
**caractérisé en ce que** sur l'axe principal (1) du dispositif est placé un dynamomètre, par exemple sous forme de tensiomètre, mesurant l'énergie sur l'axe principal (1) du dispositif entre la source dynamique (188, 189; 190) et l'arrêtoir de l'axe (125) et donc aussi les contraintes ultérieures assistées par l'arrêtoir de l'axe (125) avec les arrêtoirs de rotation (126, 129), avant leur action sur le muscle.

27. Dispositif selon la revendication 1 et l'une ou plusieurs des revendications 2 à 24,
**caractérisé en ce que** sur l'axe linéaire (132) est placé le dynamomètre qui y mesure l'énergie entre le moteur linéaire (191) ou les arrêtoirs linéaires (128, 131) et la transmission/déviation de l'énergie (133; 143, 135, 136, 137) de l'axe principal (1) du dispositif.

28. Dispositif selon la revendication 1 et l'une ou plusieurs des revendications 2 à 24,
**caractérisé en ce qu'**un potentiomètre (239) est placé sur l'axe principal (1) du dispositif ou un potentiomètre (239') avec un câble de translation (134') est placé sur l'axe linéaire (132), mesurant la position de l'axe principal (1) du dispositif et la longueur des muscles examinés ainsi que la vitesse de leur changement de longueur.

29. Dispositif selon la revendication 1 et une ou plusieurs des revendications 2 à 28,
**caractérisé en ce qu'**un accéléromètre (240) mesure au niveau du déplacement (47; 93) de la tige du pouce (14; 24) les accélérations de la contraction et du relâchement des muscles.

30. Dispositif selon la revendication 1 et une ou plusieurs des revendications 2 à 29,
**caractérisé en ce que** des capteurs électro-électroniques (204, 204' ; 205, 205') pour l'enregistrement des variations de potentiel électrique, de la température de la peau et des signaux acoustiques et des vibrations, sont mis en contact direct avec les parties du corps examinées.

31. Dispositif selon la revendication 30,
**caractérisé en ce que** les capteurs pour l'enregistrement des variations de potentiel et de la température de la peau sont des plaquettes à électrodes (204) placées dans des godets à électrodes (204') qui servent en même temps à enregistrer les moyens de contact, et **en ce que** le capteur pour l'enregistrement des signaux acoustiques ou des vibrations est un détecteur de vibrations ou un microphone (205) doté d'un tube acoustique (205'), tous deux respectivement raccordés à un dispositif électronique d'amplification et d'analyse.

32. Dispositif selon la revendication 1 et selon l'une des revendications 30 ou 31, **caractérisé en ce qu'**au moins un capteur (204, 204') et un capteur (205, 205') sont intégrés dans un boîtier (207) et disposés sur le niveau inférieur du boîtier destiné à être appuyé sur le corps, et **en ce que** le boîtier (207) est placé (242 à 246) en position mobile sur la cheville de maintien (12) ou la table d'appui pour bras (16).

33. Dispositif selon la revendication 1 et selon l'une des revendications 30 ou 31, **caractérisé en ce que** les capteurs (208, 209) pour l'enregistrement des variations de potentiel électrique et de la température de la peau ainsi que le capteur (211, 212) pour l'enregistrement des signaux acoustiques ou des vibrations sont alimentés par des ressorts compensateurs (210, 213) dans le boîtier du capteur (116), **en ce qu'**en plus le boîtier présente, sur le côté inférieur et de préférence aussi sur le côté latéral opposé à la partie du corps examinée, des surfaces d'adhérence autoadhésives (251, 252) et que des trous percés (253) mènent du côté inférieur du boîtier (216) à des raccords de tuyaux sur un appareil à vide.

34. Dispositif selon la revendication 1 et une ou plusieurs des revendications 1 à 30,
**caractérisé en ce que** lors de l'examen avec ce dispositif, on utilise une unité multisensorielle (217 à 236), dans laquelle sont intégrés, en plus des capteurs, des dispositifs de visualisation, de mise en mémoire et de traitement des valeurs mesurées.

35. Dispositif selon l'une des revendications 1 à 3,
**caractérisé en ce que** sur l'axe principal (1) du dispositif est installée à la suite des aimants mobiles (188, 189) un accouplement à glissement à la force d'accouplement (202) réglable.

36. Dispositif selon l'une des revendications 1 à 3,
**caractérisé en ce que** le levier pour le pouce (15) est relié à l'axe principal (1) du dispositif au moyen d'un boulon (203) avec position de rupture, le boulon se rompant à une valeur limite déterminée.

37. Dispositif selon la revendication 1 et l'une ou plusieurs des revendications 2 à 36,
**caractérisé en ce que** sur le levier pour le pouce (15 ; 25) est installée une échelle de mesure (99) pour le dispositif de déplacement (47).

38. Dispositif selon l'une des revendications 1 à 7,
**caractérisé en ce que** sur le petit bloc de déplacement (52) de la table d'appui pour le bras (16, 16') est installé un appareil de mesure linéaire (100) pour mesurer la position de la table d'appui.

39. Dispositif selon l'une des revendications 1 à 7,
**caractérisé en ce que** sur le petit bloc de déplacement (70) du support pour les doigts (19) est installé un appareil de mesure linéaire (104) pour mesurer la position du support des doigts (19).

40. Dispositif selon l'une des revendications 1 à 7,
**caractérisé en ce que** sur le petit bloc de déplacement (63) du support pour le bras (18) est installé un appareil de mesure linéaire (102) pour mesurer la position du support du bras.

41. Dispositif selon l'une des revendications 1 et 8, **caractérisé en ce que** sur le support des bouts des doigts (20) est installé un appareil de mesure linéaire (106) pour mesurer la position du support des bouts des doigts.
